# EUROPEAN PATENT SPECIFICATION

(11) **EP 1 850 826 B1**
(45) Date of publication and mention of the grant of the patent: **11.01.2012**
(21) Application number: 06704357.0
(22) Date of filing: 08.02.2006
(51) Int. Cl.: A61J 1/03, B65D 83/04, A61J 7/04

(54) **MEDICATION DISPENSING SYSTEM**
ARZNEISPENDERSYSTEM
SYSTEME DE DISTRIBUTION DE MEDICAMENT

(30) Priority: 08.02.2005 AU 2005900569
(43) Date of publication of application: 07.11.2007
(73) Proprietor: Katrina Susan McNamara and Paul Anthony McNamara as Trustees for the McNamara Family Trust, Sandy Bay, TAS 7005 (AU)
(72) Inventor: CANTLAY, Ian, James, North Hobart, TAS 7000 (AU); CANTLAY, Barney, North Hobart, TAS 7000 (AU)
(74) Representative: Grund, Martin
(86) International application number: PCT/AU2006/000153
(87) International publication number: WO 2006/084310

(56) References cited:
- WO-A-92/10985
- WO-A-02/078593
- WO-A1-94/15859
- WO-A1-96/19178
- WO-A1-97/43999
- GB-A- 2 199 308
- US-A- 4 572 403
- US-A1- 2004 039 481

## Description

### Field of the invention

The present invention relates to a system for dispensing medication. The system can be used for dispensing medication to patients in their own homes, nursing homes, group homes and other environments. US 2004/0039481 describes a dispensing system to still individual containers.

### Background to the invention

The use of medication to aid in patient treatment is almost ubiquitous. In most cases a patient must take several doses of medication at predetermined times. In order to achieve maximum benefit from the medication, those predetermined times allow the optimum concentration of medication to remain in the patient at all times. However, improper dosages can be detrimental to the health of a patient. Common problems encountered by health professionals are that patients often forget to take medication, they take the medication at incorrect intervals or even that they take too much in order to recover more quickly or to catch up on missed doses. These situations often mean that dosages are not optimal and therefore a longer course of treatment may be required. This may have severe consequences for the patient and/or the hospital or health institution.

There is a need in the art for a system for delivering an appropriate amount of medication at an appropriate time so that optimal dosages may be given to a patient. This is especially so for patients who are not within a health care institution where medication intake is monitored and supervised, or who are in other environments where the risk of inappropriate dosage is high.

Some existing apparatus are capable of delivering medication in pre-measured amounts at particular times. These relate particularly to fluid medicines, although there are some capable of delivering tablets at predetermined times.

However, existing systems lack flexibility, portability, and the ability to deliver complex treatment regimens. Advantages provided by the present invention will become apparent as various aspects of the invention are discussed.

### Summary of the invention

The present invention provides a system for dispensing medication to patients in their own homes, nursing homes, group homes and other environments. According to a first aspect of the present invention, there is provided a medication dispensing system including a filling apparatus, a dispensing apparatus and a portable, refillable canister and control means. The canister has a plurality of dosage compartments and is releasably mountable on the filling apparatus and dispensing apparatus. The canister also has one or more programmable devices configured to communicate with the filling and dispensing apparatus. The control means controls filling of medication into and egress of medication out of a canister attached to a filling/dispensing apparatus in accordance with information stored by the one or more programmable devices on the canister. The filling apparatus is configured to fill the dosage compartments automatically with medication in accordance with a patient's prescription communicated to the filling apparatus.

In one embodiment, the dispensing apparatus and filling apparatus are combined in a single apparatus capable of performing both dispensing and filling functions. In order to maintain conformity in the system, it is desirable that the dispensing apparatus has the same configuration as the filling apparatus.

Preferably, the control means is a computer processor attached to or contained within the filling or dispensing apparatus, and controls operation of the mechanical parts which permit filling or dispensing of medication into or from canisters attached thereto. The control means also controls transmission of information between the programmable device(s) on the canister, the apparatus and remote databases, servers or other systems with which the apparatus may share a connection.

The control means controls the dispensing apparatus to ensure that the correct dosage is dispensed at the correct time on the correct day in a dosage cycle or in accordance with a patient's dosage regimen by interrogating the programmable device on the canister. Dosage information may also be stored on the dispensing apparatus, or remotely on a database or server accessible via a communication network. This provides a medical practitioner the opportunity to tailor a dosage regimen for a specific patient and ensure that the regimen is adhered to, as well as adjust the dosage regimen remotely.

In another aspect, the present invention provides a medication dispensing system, wherein the system includes a portable, refillable canister, a dispensing apparatus, and a control means. The canister has a plurality of dosage compartments and is releasably mountable on the dispensing apparatus. The canister also contains one or more programmable devices. The control means controls the dispensing apparatus to facilitate egress of medication from a dosage compartment in accordance with information stored by the one or more programmable devices on the canister. This allows medication to be pre-filled by a pharmacy or drug manufacturer into canisters for use with the dispensing system. This is particularly suitable where the dispensing apparatus is located in a patient's residence or home environment. The dosage compartments are filled automatically with medication in accordance with a patient's prescription and medication is dispensed from the dosage compartments in accordance with information stored by the one or more programmable devices on the canister, said information pertaining to the patient's pre-determined medication dosage regimen.

According to another aspect of the present invention, there is provided a canister for use with a medication dispensing system. The canister has a shell with a dispensing opening. A dispensing wheel having a plurality of dosage compartments is rotatably couplable to the canister shell. The canister also includes one or more programmable devices configured to communicate with apparatus for filling or dispensing medication. Medication is filled into or dispensed from, the canister by attaching it to a filling and/or dispensing apparatus having a control means. The control means controls rotation of the dispensing wheel relative to the canister shell to facilitate filling of medication into and egress of medication out of the dosage compartments in accordance with information stored on the one or more programmable devices on the canister. The dosage compartments are filled automatically with medication in accordance with a patient's prescription and medication is dispensed from the dosage compartments in accordance with information stored by the one or more programmable devices on the canister, said information pertaining to the patient's pre-determined medication dosage regimen.

According to yet another aspect of the present invention, there is provided a medication dispensing system which incorporates a medication filling and dispensing device and interchangeable, refillable tamper-proof canisters. Each of the canisters has a plurality of dosage compartments and incorporated intelligence which enables the canisters to carry information relating to medication doses and/or a medication regimen, including time for medication and patient information, Information carried on the canister can be relayed from one filling/dispensing apparatus to another facilitate dispensing or filling of medication in accordance with a pre-determined dosage regimen.

Each dosage compartment in the canister holds a dose of medication until it is due to be dispensed. The canister is removable from one dispensing and/or filling apparatus, for installation to another dispensing and/or filling apparatus. Accordingly, canisters can be filled with medication at a first location, and the medication may be dispensed at a second, third or fourth location which may include where the patient resides or is being treated. Hence the medication dispensing system is portable.

For example, following treatment in hospital a patient may be discharged but still require a course of medication to complete the treatment. Thus the patient may be discharged with a canister containing the required medication or a prescription(s) which can then be filled into a canister at a pharmacy or the like. The medication can then be dispensed from the canister using a dispensing apparatus located in the patient's residence (or other home environment) without the need for constant direct monitoring by hospital staff. The dispensing apparatus may be loaned to the patient by the hospital, leased, purchased or provided under some other plan.

The system will only allow medication to be dispensed when a set of predetermined conditions have been satisfied. These conditions may include but are not limited to the identity of the patient or person accessing the canister being confirmed, the time of day medication is to be dispensed is checked, the actual time of day corresponds to the necessary dosage time, and other aspects of the patient's dosage regimen being compliant, as may be determined by a medical practitioner.

Each canister includes at least one programmable device which allows the canister to be tracked through its cycle of filling, transit from the hospital pharmacy to the patient, dispensing to the patient and return to a hospital, pharmacy or other repository. The programmable devices may include, but are not limited to, radio frequency identification devices (RFID), microchips, subscriber identity module (SIM) chips, smart card technology or the like, or other legacy technologies, such as barcodes or magnetic strips. The programmable device allows the control means to monitor and/or regulate dispensing of medication in a patient-specific manner, to monitor and record dispensing and tampering events, and the identity of the patient receiving the medication for example.

Advantageously, by incorporating such intelligence on the canister, information relating to the patient and their medication can be transported with the canister containing the medication, and is not required to be stored centrally on a database or stored on a filling or dispensing apparatus. This means that medication can be dispensed from the canister when it is fitted to any dispensing apparatus, or replenished when it is fitted to any filling apparatus, without the dispensing/filling apparatus being pre-configured for a particular patient's dosage regimen, or the need to make a connection to a remote database. However, it is to be understood that the information on the canister can be updated via such connections, when changes to a prescription are made for example. It is envisaged that each canister contains sufficient dosage compartments to fill a dosage cycle. Alternatively, multiple canisters may be required to fulfil a dosage cycle.

Preferably, the canisters are tamper-proof, and are fitted with tamper-prevention means to stop unauthorised access to the medication inside the canister. Preferably the tamper-prevention means includes, in one form, one or more solenoids to prevent the dispensing wheel from rotating within the canister shell when the canister is not attached to a dispensing apparatus. Thus, the solenoid requires a signal and/or power from the apparatus to release the dispensing wheel. The canisters may also include tamper-detection means for detecting tamper attempts. One example of tamper-detection means is a contact switch located between a faceplate and shell of a canister. When a knife or other implement is inserted between the faceplate and the canister shell in an attempt to pry them apart, the contact switch generates a signal which is detected by the programmable device on the canister as a tamper attempt.

Preferably, programmable device(s) on the canisters monitor tampering attacks on the canister, both successful and unsuccessful. In this regard, the programmable device may have a tamper-detection status storage component for storing information relating to a tamper attempt and a tamper log, to record changes in the tamper-detection status. In a preferred embodiment, when a canister is fitted to a filling/dispensing apparatus, the programmable device on the canister communicates to the dispensing/filling apparatus (or a remote service) detection of any tamper attempt which has been detected on the canister.

The canisters can be attached to the dispensing/filling apparatus at various locations for either dispensing or filling. In use, a canister may be filled in one location, such as a pharmacy, then transferred to another location for dispensing, such as a patient's residence. Preferably, when filled, the canister is given an identifier which is cross-referenced with the patient's identity to ensure the medication in the canister is dispensed to the correct patient.

Preferably, the system further includes a printer for printing a label attachable to the canister. Such labels should identify the patient and dosage information relating to the medication which has been prescribed to the patient. Preferably, the label is affixed to a portion of the canister where it will not be removed easily or destroyed, for example on the inside of the canister shell, where the shell is transparent. Such positioning may be automatic, using a position locating aid such as a window in the canister shell to locate the correct label position. It is desirable that the label is re-printed each time the patient's prescription is changed. This may occur automatically. The label is useful for quick reference by a pharmacist or physician responsible for the patient's medication regimen. The label may include a barcode or electronic display such as a robust digital alphanumeric LCD or LED.

The dispensing system may also include a movable (vibrating) member configured to move dispensed medication so that individual medication units can be separately identified by an optical detection device. That is, the movable member may vibrate or otherwise agitate tablets, capsules or the like so that any units of medication which are on top of one another or otherwise obstruct direct view by an optical detection device can be separated and identified. The movable member may be incorporated into a platform of the dispensing apparatus, where an optical detector (such as a camera) uses image recognition algorithms to validate a dispensed dosage of medication. The moving member may also be incorporated into the filling apparatus as part of the filling meter to ensure that drugs filled into the canister are accurately counted. Such moving member would be software controllable and may, for example, agitate medication units in response to an error signal received from the optical detection device.

Upon attachment of the canister to the dispensing apparatus and satisfying any predetermined rules, e.g. that the medication is being dispensed to the correct patient, the apparatus dispenses a predetermined dosage of medication to the patient. In scenarios where a patient's carer attaches the canister to the dispensing apparatus (e.g. where a patient is bed-ridden) the carer's identity may be confirmed instead of that of the patient. The patient may have a smart card, coded band or other device which may be used to identify the patient/carer. For sophisticated dispensers, biometric data such as fingerprint and eye scans may be used to verify the identity of the individual to whom the medication is to be dispensed. In a preferred embodiment, the identity of the person to whom the medication is dispensed and the time at which dispensing occurs is recorded either by the apparatus, remotely or by the programmable device on the canister, or by a combination of these.

Tamper-prevention means incorporated into the canister may include conventional detection systems which use closed/open circuits, magnetic field detectors, impedance monitors and the like, and may be lockable (eg by key coded RFID) thereby restricting unauthorized access. A power supply (e.g. battery, capacitor or other device) may be included in the canister to power a clock and facilitate recording of tamper detection events and times on the programmable device on the canister.

Preferably, the programmable device monitors all tampering attempts, both successful and unsuccessful. In one embodiment, the tamper log is accessible by a central administrator, and/or a periodic report may be generated. It is desirable that any successful tampering event is reported to a central administrator or other authorised entity as soon as practicable after detection of the tampering event.

The control means uses information from the programmable device on the canister and the patient's medication requirements (i.e. prescription which is preferably also stored on the programmable device on the canister) to correctly dispense the medication. Use of such information should result in timely dispensing of medication, prevent dispensing of medication for which the patient has a contraindication and preferably raise an alert for the patient, physician or carer, should a contraindication arise.

A canister can be recognized by and operate with numerous dispensing and filling apparatus. Information stored by the programmable device is therefore transferable, with the canister, from one location to the next. The canisters are configured to ensure the correct dosage is dispensed at the correct time once fitted to any apparatus.

In one of its forms, the present invention allows the canisters to act as mobile access to a patient's prescription(s). One patient may have a number of canisters to suit his or her dosage regimen. Advantageously, where the dispensing/filling apparatus has a connection to a central server the regimen can be changed centrally when a canister is attached to a dispensing/filling apparatus with no interruption to the patient and potentially without the need to discard medication.

The canister may have more than one set of dosage compartments thereby allowing a single canister to deliver complex dosage regimens. In a preferred embodiment, canisters are round with an outer rim configuration in which a set of dosage compartments can be arranged. Alternatively, multiple canisters may be fitted to a single filling and/or dispensing apparatus, thereby allowing complex dosage regimens to be accommodated, and allowing a single apparatus to deliver medication to a number of patients, e.g. in a nursing home or hospital ward.

In one embodiment, each dosage compartment in a canister has its own programmable device. These devices can store information relevant to the contents and position of individual compartments, relative to a dispensing opening in the canister. This allows greater control over the regulation and monitoring of dosages introduced into each dosage compartment as well as dosages dispensed to the patient. In such an embodiment, it is envisaged that each dosage compartment has an allocated RFID tag or other programmable device.

Thus, the present invention provides canisters which are "smart", with the capacity to store and communicate information pertaining to- the -patient and their medication regimen including the identity of the patient to whom the canister belongs, as well as the time of any tamper attempts. The information stored on the canister may include one or more of the following: the dosage in each compartment, the time of day the dosage should be dispensed from each compartment, which compartments have dispensed their dosages and, at the end of the canister's dosage cycle, which dosages remain undispensed from the canister.

The intelligence of the canister is intended grow with the further development of smart card, RFID and other such technologies, with the unique integration of those technologies applied to the canisters and their interaction with technology in the dispensing/filling apparatus.

The canisters are designed to be patient specific and, using reprogrammable RFID and Smart Card technology, provide a premium dispensing service to patients in their residences and other care environments. Each patient may be allocated one or more canisters depending upon their dosage regimen. In some circumstances, it may be desirable for a patient to have up to four canisters moving in the system at any one time. This allows flexibility to accommodate filling of canisters under prescription at pharmacies, transit from pharmacies to patients, dispensing and potential overlap of dosage cycles.

In addition to regulating dispensing of medication, it is desirable that the control means also controls the introduction of medication into the canister during filling. That is, a dose of medication may be controllably introduced into a dosage compartment automatically using the filling apparatus under the control of the control means. In order to allow ingress of medication, a filling meter and/or measuring means may be required. The filling meter preferably identifies a drug prior to its introduction into a dosage compartment. The filling meter may measure or count tablets, capsules or other dry medication prior to release into the relevant dosage compartments. In addition to filling which takes place at pharmacies and drug manufacturers, the canisters may also be filled *in situ,* for example at home, a nursing home or in respite care by a visiting pharmacist or similarly qualified person or organisation. Any medication contained in the filling apparatus and/or the filing meter should be securely stored and accessible only by the (visiting) pharmacist or other authorized person.

The apparatus may operate with the canister in a flat (horizontal) or upright (vertical) orientation for filling, and in an upright position for dispensing. In the upright orientation, the canister need not be opened for filling. In the horizontal orientation, the canister should be opened to reveal the dosage compartments therein. This also makes possible insertion of dispensing wheels having differently configured dosage compartments for multiple dispensing regimens, and cleaning when required.

Using the present invention, a single apparatus can fill canisters and dispense medication for multiple patients whilst maintaining the security of each patient's medication and the confidentiality of each patient's personal and medical information. Moreover, a single canister can dispense medication from or be filled by any dispensing/filling apparatus in the system since information required to control release of the medication is stored on the canister itself. The canisters may be configured to give the patient access to the personal and medical information stored on the canister, or authorise access by a healthcare professional or other designated person.

It is desirable that certain embodiments of the dispensing and/or filling apparatus include devices which allow it to communicate with external devices, such as a central (remotely located) computer which is part of a communication network. In particular embodiments the dispensing and/or filling apparatus may have wireless connectivity permitting the above-mentioned monitoring and security functions without a direct physical link to the computer. Alternatively, the computer may be physically linked to the apparatus via a wired network such as the Internet, telephone network, or other communications network. Further, it is desirable that the programmable identification device on the canister is configured for wireless tracking. RFID is one suitable means for providing wireless identification and tracking. Smart Card technology may be similarly suitable.

In embodiments where the dispensing and/or filling apparatus is configured for communication with a remotely located server, the apparatus can receive and transmit information relating to the patient, a dosage regimen, medication, tampering and other information relevant to dispensing of medication to a patient. Centralized dissemination- of dosage information provides an auditable trail as to the quantity and kind of drugs filled into and dispensed from canisters, the identity of patients receiving each dose and in some cases, the identity of carers dispensing a dosage on a patient's behalf.

The apparatus may also have a monitoring function that allows for monitoring drugs loaded into a canister (and compartments thereof), dispensing of drugs, the time and location of loading and dispensing and an identifier corresponding to the individual instigating the loading and/or dispensing. In one embodiment, the dispensing and/or filling apparatus may also include a web browser to communicate with authorised remotely located parties such as pharmacists and physicians over the Internet.

In many cases, the dispensing apparatus will be a different apparatus to the filling apparatus and a plurality of dispensing apparatus may be used over time. The apparatus may also accept information from other approved sources such as a health care facility's computer system or a pharmacy. Such information may also be stored by technology on the canister, thereby allowing the information to be transferred to any apparatus to which the canister is attached. The apparatus may therefore relay dosage information to the programmable device on the canister at the time of filling or dispensing.

Preferably, the filling apparatus includes a filling meter for measuring a quantity of drugs to be filled into a dosage compartment of a canister fitted to the apparatus. Used in concert with the monitoring of medication using the programmable devices on the canisters as well as smart card or other technology for personnel identification, the filling meter provides a useful link in the medication dispensing system for audit purposes and potential modification of drug handling procedures in pharmacies and hospitals.

Information required for correct dispensing of medication from a canister is stored, at least in part on the canister itself and the apparatus uses that information to dispense medication appropriately. Accordingly, dispensing of medication can occur without the need to interrogate the centralised database on every dispensing event. It should be noted, however, that a connection between a canister and a centralised computer may be made from time to time to ensure that the dosage regimen is being adhered to, to effect changes to the regimen where necessary and for reporting, billing and the like.

Connection to a centralized computer allows immediate centralised updating of patient information and altering of a dosage regimen if necessary. Meanwhile, storage of dispensing information on the canister reduces bandwidth requirements of the system since a connection between the apparatus and the centralised database is necessary only on a periodic basis. Thus, transmission of data between the canister and the central database, via the apparatus, takes place in batches (e.g. at the end of every day, week or month, for example). Interim storage of such information on the apparatus may be desirable.

Preferably, the canister and/or the apparatus further includes an alert system. The alert system may warn a patient when a dosage is due to be dispensed, and may raise an alarm when a patient has not dispensed a prescribed medication from the canister in accordance with the predetermined dosage regimen. This may occur immediately after the predetermined dosage dispensing time has passed, or after a pre-determined time frame has elapsed. There may be a plurality of time frames and corresponding alert levels, where elapsing of a longer time frame escalates the alert to a higher level. Alternatively or additionally, the alert system may be operated by the patient to indicate that the patient has fallen or requires some form of assistance. This may trigger automatically a call to an emergency service (e.g. ambulance), healthcare organisation, relative or carer for action.

The dispensing system may also include an automated billing interface.

In such an embodiment, the dosage and filling information is used to update a patient's billing records and used by a pharmacy or the like to bill automatically the patient or relevant agencies such as Medicare, a healthcare or insurance provider, employer or other regulatory body, organisation or billable entity for drugs dispensed. Billing information can also be used by the Pharmaceutical Benefits Scheme (PBS) or similar body, to formulate budgets and research drug usage patterns, whilst observing privacy laws to protect the personal information of patient using the system.

The interconnectivity of the system allows a dispensing apparatus to transfer relevant information to and/or from an information system in a care facility, in the patient's home or elsewhere. Preferably, access in the patient's own home would be provided via a dispensing apparatus purchased, on loan or leased for that residence. Preferably, the in-home dispensing apparatus has access to a remote server by way of phone line, Internet, cable, wireless or other connection.

The patient's in-home apparatus may also be connectable to a printer for updated drug information printout via the phone or similar communication service. The apparatus may also have a display such as a LED, LCD or other screen to display information to the patient, their carer, pharmacist, doctor or the care staff during boot-up, filling, dispensing, data transfer or reporting using the apparatus.

In order to allow filling and/or dispensing functions, the apparatus has one or more dispensing zones. An example of such a dispensing zone is an automatic hinged gate arrangement. In the case where a canister has more than one set of dosage compartments, the apparatus may require a plurality of dispensing zones, such as independently operable gates to allow controlled medication ingress (filling) and egress (dispensing). The specific arrangement of gate(s) will depend upon the arrangement of the canisters (and particularly dosage compartments within the canisters) being attached to the apparatus.

The apparatus may be configured to dispense dosages from a canister having a single dispensing wheel with one dosage compartment per day, or using a canister having a plurality of dosage compartments per day. In the latter arrangement, greater flexibility in dosage regimens is provided when the apparatus is coupled with a multiple gate array.

The dosage regimen is regulated by a control means in conjunction with the programmable device(s) on the canister. As discussed above, the canister and optionally each of the dosage compartments in the canister have programmable devices. These devices permit identification and storage of dosage-related information including the identity of the canister, a number of outer rim sections, a number of dosage compartments, a medication or plurality of medications contained within the dosage compartments, quantities and/or combinations thereof, the time at which a dosage was dispensed or is due to be dispensed from a compartment, and the identity of a patient to whom the canister belongs.

Since drug dispensing and tamper-detection information is recordable by the technology built into the canister and optionally the dispensing system, patients receiving medication using the-inventive system are able to move between different care environments whilst automatically maintaining and adhering to their prescribed dosage regimen. For example, a patient at home may have a personal dispensing apparatus which, in its simplest model, can be utilized only to dispense drugs.

By incorporating various features, preferably in the form of modules such as displays, alarm modules, transmitters and receivers, optical detection devices and the like, the apparatus may also accommodate remote monitoring, provision of dosage reminders and emergency alert services. The system may also update in real-time, drug information to a patient's home via a telephone, Internet, wireless or other service. The apparatus can be configured to communicate with the patient or other designated contact by audible messages, SMS, mobile phone voice message, or other communication means to remind a patient to dispense medication and/or take dosages that have been dispensed from the canister in advance of the scheduled dosage time. The canister itself may also be configured to make an audible and/or visible and/or tactile (e.g. vibrating) cue to remind the patient that a dosage is due to be dispensed.

### Brief description of the Drawings

The present invention will now be described in greater detail with reference to the accompanying drawings. It is to be understood that the specificity of the embodiments illustrated and described does not limit the generality of the preceding description of the invention or its scope and applicability.
Figures 1 and 1 A illustrate a canister shell according to an embodiment of the present invention.
Figure 2 illustrates a dispensing wheel to fit within the canister shell shown in Figure 1. Figure 2A shows an alternative embodiment of a dispensing wheel having two outer rims.
Figure 3 is an exploded view of a canister shell axle with a spring and washer arrangement according to an embodiment of the invention.
Figure 4 is a top view of a faceplate for covering dosage compartments in a canister according to an embodiment of the invention. Figure 4A is a side view of the faceplate of Figure 4.
Figure 5 illustrates the underside of a possible configuration of a drive clamp that faces the canister shell and interlocks the drive shaft with the dispensing wheel. Figure 5A is a side view of the drive clamp of Figure 5. Figure 5B is a top view of the drive clamp of Figures 5 and 5A.
Figure 6 shows a dispensing/filling apparatus according to an embodiment of the present invention.
Figures 7 and 7A illustrate an example of holding lugs for retaining a canister on the apparatus, according to an embodiment of the present invention.
Figure 8 illustrates a filling meter for attachment to a filling apparatus according to an embodiment of the present invention.
Figure 9 is a perspective view of hinged gates on a dispensing apparatus and/or filling apparatus according to an embodiment of the invention. Figure 9A is a side view of the hinged gates of Figure 9.

### Detailed description

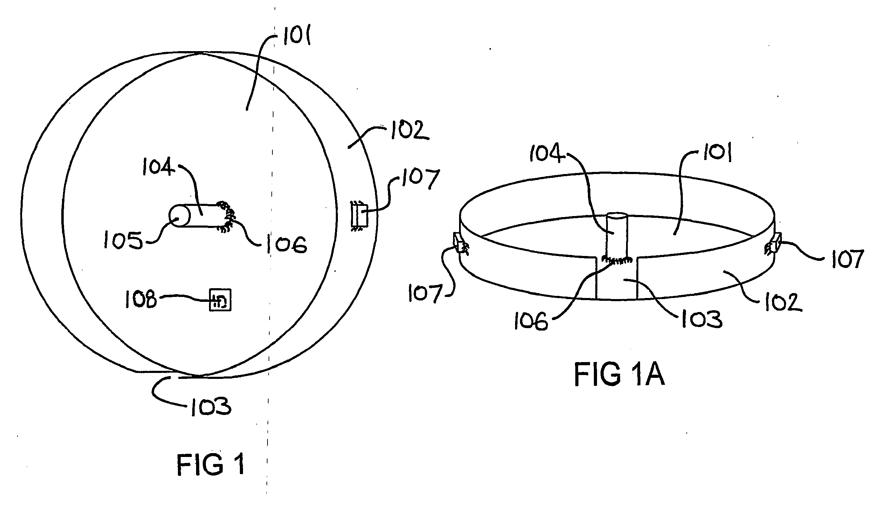

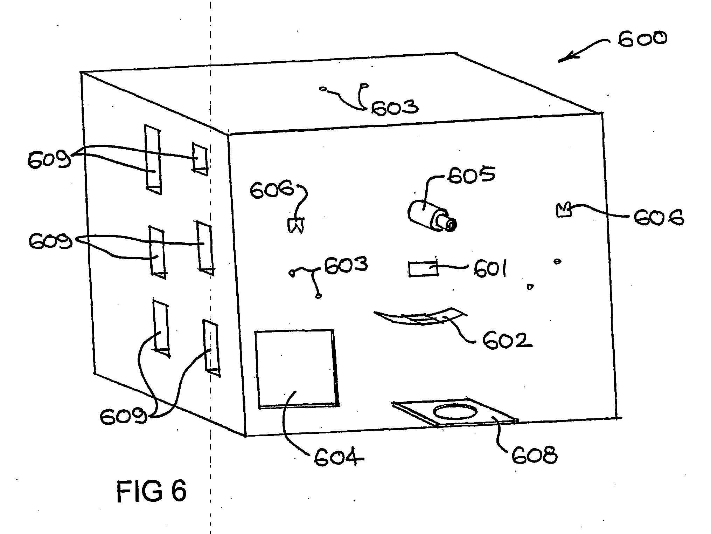

Referring firstly to Figure 1 an example of a canister shell 101 is shown for use with an embodiment of the present invention. Canister shell 101 fits on the front of a dispensing/filling apparatus 600 (Figure 6), hereinafter referred to as "apparatus". The canister shell (and other parts of the canister) may be made from any suitable material, such as a clear polycarbonate plastic or other suitable food-grade plastic. It may be fabricated by injection moulding or another suitable method.

A rigid outer wall 102 with an opening 103 for dispensing drugs is provided around the periphery of the canister shell 101. A rigid hollow axle 104, having a threaded interior 105 for part of its length allows attachment of the canister to the apparatus. A set of rigid teeth 106 are moulded around the end of the axle 104 to prevent manual rotation of the dispensing wheel once fitted to the apparatus.

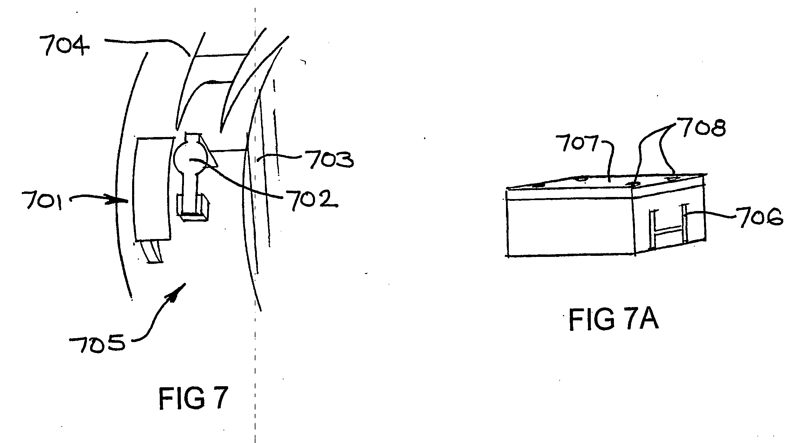

Moulded holding lugs 107 and spring pin slots and blocks are provided on opposing sides of the outer wall 102. The holding lugs, pins and blocks may be located at other points on the outer wall and may be positioned so as to interlock or interact with hinged gate(s) on the apparatus. A possible configuration of the holding lugs with spring pins is shown in Figures 7 and 7A and are referred to in greater detail below.

For delivery of medication, the canister is attached to the apparatus preferably with the delivery opening 103 at the lowest point for dispensing drugs using gravity. Alternatively, the canister may be inverted with the delivery space at the top for filling either manually or using an automated filling attachment or meter which can be fitted to or incorporated into the filling apparatus.

The canister includes one or more programmable devices such as a radio frequency identification device (RFID), microchip or smart card 108 to track and monitor the canister and medication dispensed from it and to keep a record of tamper attempts made on the canister. The use of the programmable device(s) attached to the canister shell 101 in Figure 1 is illustrative only. Preferably, the programmable device 108 is embedded in the canister in such a way that it is not easily removed or tampered with. In a particularly preferred embodiment, a plurality of identification devices is incorporated into the canister so that individual dosage compartments in the canister can be separately monitored and tracked. This may include monitoring the status of each dosage compartment (e.g. empty/full) and the position of a dosage compartment relative to the canister shell opening 103, for example.

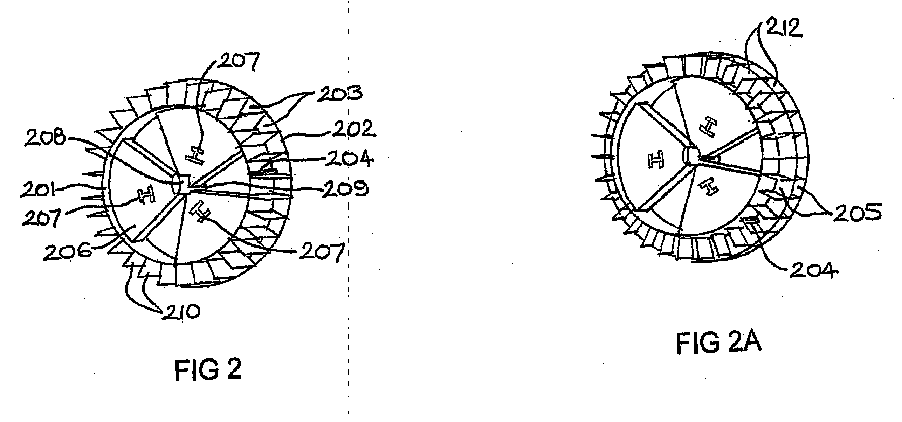

Figure 2 illustrates an example of a dispensing wheel designed to fit within the canister shell shown in Figure 1. Figure 2A shows an alternative embodiment of a dispensing wheel having a different dosage compartment configuration provided by two outer rims. In the embodiments illustrated, the dosage compartments are provided by way of a spoked wheel that fits into the canister shell, inside the outer wall 102.

Referring in particular to the embodiment illustrated in Figure 2, the dispensing wheel has an inner body 201 and an outer rim 202 in which dosage compartments 203 are defined by spokes 210. The outer rim 202 is preferably detachable from the inner body 201. This facilitates easy cleaning and replacement with an outer rim having a different dosage compartment configuration, such as the one illustrated in Figure 2A. The outer rims 212 illustrated in Figure 2A provide two adjacent sets of dosage compartments 205 which extend in parallel around the dispensing wheel.

Preferably, the outer rim 202 has slots to accept ridges 204 on the inner body thereby preventing independent slippage between the two parts. In order to provide a conveniently portable canister, it is envisaged that a suitable size for compartments on a single fitted outer rim would be in the order of 15 cubic centimetres, although this is a guide only.

The configuration illustrated in Figure 2 has 29 (4x7+1) compartments to accommodate up to 4 weeks of dosages. Preferably, canisters have up to 32 compartments which are shaped so as to avoid tablets or capsules jamming. The canister may be loaded with dispensing wheels having outer rims with different configurations, with a plurality of compartments of reduced size occupying the same volume as a single compartment in wheel 205 (see Figure 2). These separate outer rims may operate with a plurality of coordinated delivery gates provided on the dispensing apparatus, so that synchronization between the delivery space in the canister shell and the delivery gates permits egress of a dosage from an individual dosage compartment (see Figure 11). Separate outer rims 205 may be loaded onto the inner body 201 and filled one at a time in sequence. Alternatively, they may be filled automatically through the delivery space when the wheel is in an inverted filling position.

Preferably, the dispensing wheel has at least one spare compartment which is intended to remain empty to provide the canister with a rest position in which the empty compartment is aligned with the delivery space. It is preferred that the canister automatically assumes the "rest position" prior to the canister being removed from a filling or dispensing apparatus, to prevent uncontrolled loss of medication from the canister in transit. In one mode of operation, the wheel returns to this rest position after each dosage delivery.

The inner region of the dispensing wheel may be a solid piece or split into a plurality of spokes, to suit various design principles and to incorporate other components such as batteries, identity tags and electronic components for tamper proofing and other functions. The centre portion of the wheel in Figure 2 has three large spokes 206 although this configuration is only one of many possible configurations.

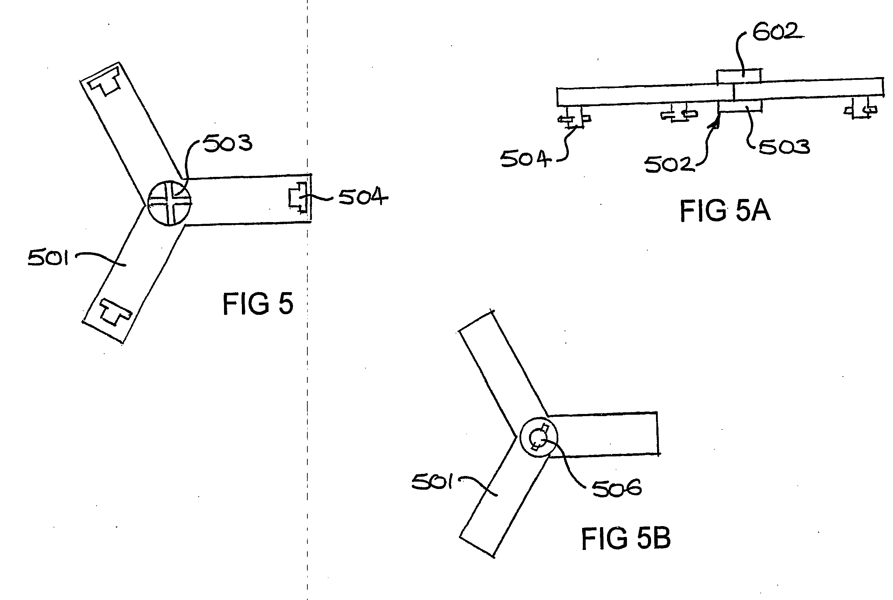

Located in the spokes are slots 207. These slots 207 are shaped to receive pins 504 of a drive clamp 501 of the apparatus (see Figure 5), and are also shaped to lock the clamp in position. At the centre of the dispensing wheel is a hollow sleeve 208 which has rigid teeth 209 at the back to interlock with teeth 106 on the canister shell.

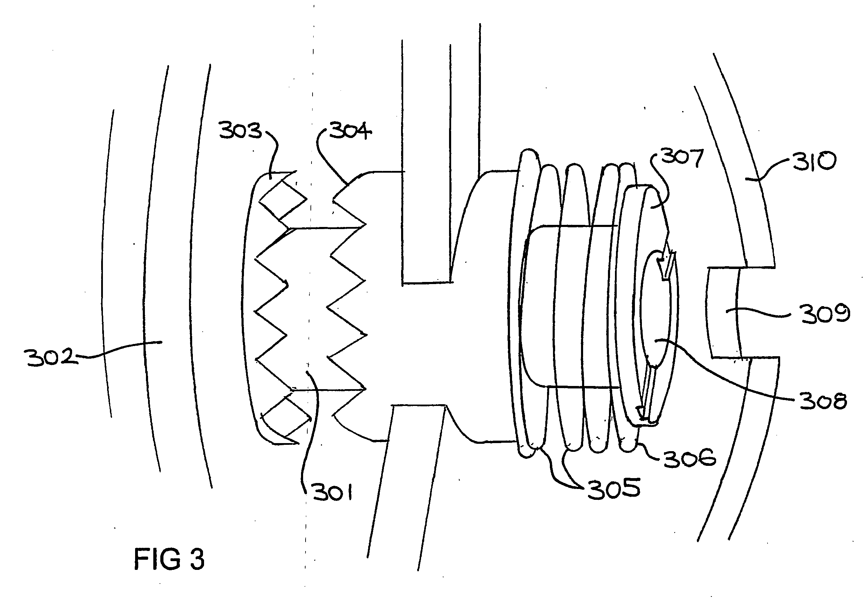

Figure 3 is an enlarged and exploded view showing parts of a canister and dispensing wheel near the hollow axle. A sleeve 308 which is shorter than the axle 301 of the canister shell 302 fits -over the- axle 301 -with sufficient clearance to allow it to turn but without allowing lateral movement against the axle. The wheel may have a bearing used between it and the axle. A locking nut 307 is provided to hold the spring and washer arrangement in position. Locking nut 307 has an outside thread and screws into the threaded interior of the axle on the canister shell although it is to be understood that other arrangements may be equally effective. Locking nut 307 also has a hollow interior 308 providing a clear passage between the canister shell 302 and the dispensing wheel where an insert 309 from drive clamp 310 (see also Figure 5) can be inserted. This allows the drive shaft from the filling/dispensing apparatus to extend through and interlock with the insert 309 from the drive clamp enabling the dispensing wheel to interact with the drive shaft.

Teeth 404 are provided at the back of the wheel and interlock with teeth 403 of the canister shell (see also teeth 106 in Figure 1). When the teeth on the dispensing wheel and the canister shell are interlocked the dispensing wheel cannot be turned independently of the canister shell and medication can not escape from the canister.

The hollow sleeve 308 is slidably engaged onto the axle 301 of the canister shell and a spring and washer configuration 305, 306 fitted to force the teeth 304 on the dispensing wheel to interlock with the teeth 303 of the canister shell. The force of the spring 305 should be sufficient to resist force applied in either a pulling or rotational motion in an attempt to separate the dispensing wheel from the canister shell and gain access to the contents of the canister. During operation, to facilitate separation of the canister and dispensing wheel, the force of the spring is overcome by a force which is controllably applied by the apparatus. In this sense, the canister is tamper-proof. A bearing may also be provided between the dispensing wheel and the axle.

Independent rotational motion between the canister shell and the wheel contained therein is also countered by spring pins that are located in the holding lugs 107 of the outer rim of the canister shell. The holding lugs 107 and pins may be positioned at other points on the canister shell and may be positioned so as to interlock or interact with the hinged gate(s) on the apparatus. A possible configuration for the holding lugs with spring pins is illustrated in Figures 7 and 7A. Alternatively, the spring arrangement may be substituted by, for example, an electrically operated solenoid which locks the dispensing wheel from rotational movement. Upon entry of a code the solenoid can be released by application of a current from the apparatus, through electrically conductive parts of the drive shaft to the canister.

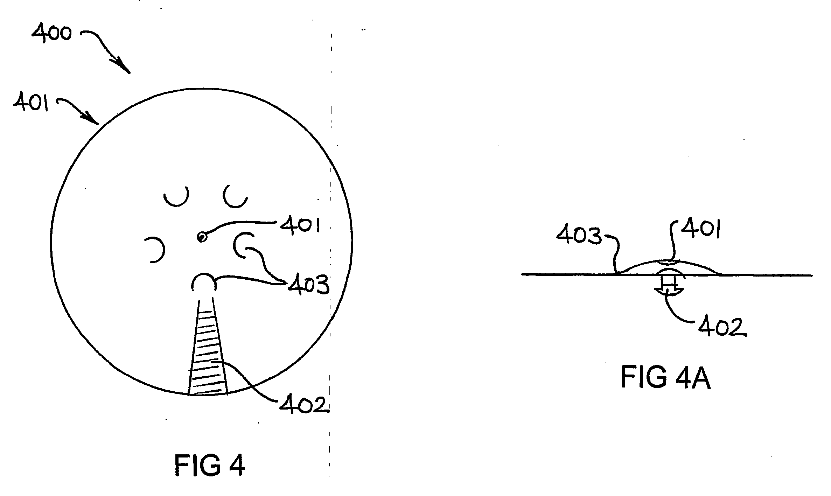

Now referring to Figures 4 and 4A, there is shown a faceplate which covers the dispensing wheel when applied to the canister shell. The faceplate, when attached, may be slightly shallower than the side wall of the canister shell even when the canister is in an active position on the apparatus. This depth differential ensures that the dosage compartments in the wheel do not extend beyond the side wall of the canister shell thus precluding dosage spill or tampered access. Additionally, this discrepancy in height mitigates against levers being inserted between the outer rim, canister shell and faceplate and thereby provides a further level of tamper protection.

Preferably, the faceplate 400 has gripping slots 403 configured to receive the thumbs and fingers of a user to hold the canister. However, they do not permit the user to generate enough force to turn the faceplate and gain access to the inside of the canister. This configuration is intended to mitigate against tampering.

The faceplate 400 is shown with a visual positioning aid 404 to facilitate easy aligning of the faceplate with the canister shell for re-attachment, eg. after canister filling or cleaning. It is transparent and allows visual inspection to check that the dispensing wheel and faceplate have returned to a rest position on the apparatus before the canister can be removed from the apparatus.

Faceplate 400 also has a keyhole 401 which enables a locking mechanism to be secured in the locking slot on the topside of the drive clamp. The locking pin 402 in Figure 4A may vary in shape and size and the locking mechanism may be configured to operate mechanically and/or or electronically. The locking pin 402 may be manufactured from metal or plastic. The keyhole may include a coded slot or other secure configuration.

Now referring to Figure 5, the underside of a drive clamp is shown. The drive clamp faces the canister shell and interlocks the drive shaft of the apparatus with the dispensing wheel of the canister. The centre of the drive clamp body 501 has an insert 502 (see also 309 in Figure 3) that fits inside the hollow of the locking nut which in turn screws into the interior of-the canister shell axle. This insert has a shaped interior 503 that mates with the end of the drive shaft to interlock. This shaped interior may be plastic or metal or composed of another suitable material. Where the material is an electrical conductor, it may also extend through the body of the clamp to interact with a faceplate locking slot. This enables use of electrical current to control engagement and disengagement of the drive shaft with the dispensing wheel and can facilitate communication between the apparatus and programmable devices on the canister. Thus electrical current can pass from the drive shaft of the apparatus to the canister through the conducting interior of the clamp body insert 502. This current may also be used to enhance the security of the canister by way of an electronic locking mechanism. The accompanying drawings do not show electronic circuitry in the canister or filling/dispensing apparatus since it can be designed using standard techniques known to the person skilled in the art.

In the embodiment illustrated, the drive clamp body 501 has three wheel locking pins 504 which fit into the mating locking slots 207 on the dispensing wheel to secure the drive clamp to the wheel. The configuration and shape of the locking pins may take any suitable form. The one shown is for illustrative purposes only. The pins are of sufficient dimension to withstand shear and torsional forces resulting from operation of the apparatus or other applied forces. In the embodiment illustrated, the drive clamp is integral to the operation of the system, providing driveability and connectivity between the apparatus and the canister, although other drive arrangements may be used.

Figure 5B illustrates the top of the drive clamp illustrated in Figures 5 and 5A. The top of the drive clamp has a locking slot 506 which mates with the locking pin mechanism from the faceplate (see Figure 4). The locking slot 506 allows the faceplate to be locked in position with respect to the dispensing wheel so as to permit rotation of the two parts together. The locking slot 506 enables the faceplate to be secured in position and only removed by use of a suitable implement (key) or entry of a security code.

The locking slot 506 may be formed in plastic, metal or any other suitable material. Where the locking slot is lined with an electrical conductor electrical current can flow from the apparatus through the drive shaft to the faceplate and other components of the canister. Such current may be utilised to transfer data between the canister and the apparatus.

Figure 6 is a simplified illustration of a dispensing/filling apparatus 600 according to an embodiment of the present invention. When fitted to the apparatus, information carried by the programmable device on the canister is read by the device reader 601 on the apparatus. The device reader is configured to communicate with the programmable identification device which may include one or more of a RFID device, a microchip, a subscriber identity module (SIM) chip, a smart card, smart chip or other electronic device. The device reader may also be configured to read barcodes or magnetic stripes on the canister.

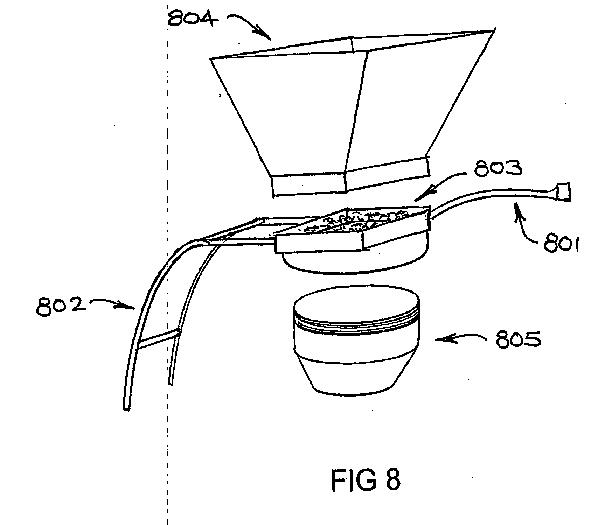

The embodiment illustrated in Figure 6 shows the apparatus in an operating configuration in which medication can be dispensed through the delivery space of the canister shell when a canister is fitted to the apparatus with its delivery space at the lowest point. A dispensing zone is provided in the form of a hinged gate 602 has a centrally located raised portion which, when the canister is fitted to the apparatus, fits into the canister shell opening (delivery space) 103. The thickness of the raised portion corresponds with the thickness of the side wall of the canister shell. Gate 602 may be a unitary operating gate or it may include more than one independently operable gate portions to accommodate a canister having two or more outer rims (sets of dosage compartments) fitted to the dispensing wheel. Figure 8 shows a dispensing zone having a twin gate arrangement.

Alternatively, a canister can be fitted to the apparatus with the delivery space opening upward. In this configuration the canisters can be filled manually through the delivery space or semi automatically using a filling meter (Figure 8) to load automatically the correct dosages of medication into the dosage compartments. The filling meter interacts with the apparatus via control means which also controls the drive shaft to rotate the dispensing wheel to the appropriate location for a dosage to be filled into a dosage compartment.

Referring now to Figure 7, holding lugs 701 on the apparatus retain the canister against the pressure exerted by the apparatus to compress the spring in the canister. The lugs cover pins 702 which are preferably made of spring metal. These pins fit through slots in the side wall of the canister-shell in such a way that the dispensing wheel is precluded from rotation. These pins also add to the tamper-proofing provided by the spring forced interlocking teeth of the wheel and the canister shell. The pins 702 have split ends that slide on either side of a dosage compartment wall thus preventing manual rotation in either direction. The pins have a shaped metal configuration with wings extending laterally and are kept in position by the spring action of their metal construction.

The pins may be lifted out of the locking position using specially shaped wedges 704 fitted to the apparatus as part of the lug holders. The wedges fit under the wings of the pins when the canister is rotated into position on the apparatus. The wedges 704 are of sufficient dimension to lift the pins 702 clear of the dosage compartment walls but not withdraw them fully from the side wall of the canister shell. The side wall is constructed with a raised recess to facilitate this. Preferably, the pins have rolled ends, which prevent them from being withdrawn through the side wall of the canister shell.

The holding lug 701 may have a shaped entry to receive the lifting wedge. The shaped entry may also prevent other tools from being inserted to raise the pins. The lugs 701 may be of two part construction, with the base 706 being moulded to the side wall 705 of the canister shell and the top 707 attached to the base 706 by fasteners 708. However, it is to be understood that this is only one possible configuration for the lugs 701 and pins 702 and other configurations are possible within the scope of the present invention.

The spring pins are positioned to interact with the walls of the dosage compartments of the dispensing wheel. The pins may require a separate spring to hold them in position. For egress of a dosage, the delivery space 103 of the canister shell is aligned with an individual dosage compartment 305. The delivery space 103 also aligns with the hinged gate(s) of the apparatus.

It is to be understood that other, preferably electronically operable arrangements can be used as an alternative to the spring pins illustrated. For example, the spring pins could be replaced with a solenoid having electronically controlled pins which, under the control of the control means, slide into the slots in the canister shell to prevent manual rotation of the canister when fitted to the apparatus.

Similarly, the faceplate which covers the dosage compartments in the canister can be controllably released by electronically actuated solenoids mounted on the canister. In such an arrangement, when the canister is attached to a filling or dispensing apparatus, the control means can communicate with each of the solenoids electronically. This enables the control means to monitor and record each canister access event (including dispensing, opening and tamper detections) and maintain a record on a remote or local storage device, and/or on the programmable device on the canister.

When the canister is not attached to the dispensing apparatus the solenoids cannot be activated because they rely on both power from the apparatus and the coded information available only on a dispensing apparatus. Thus the tamper proofing of the canister is maintained at maximum levels to allow for secure transport from, for example, a pharmacy to the patient. A power supply (e.g. battery, capacitor or other device) is preferably included in the canister to operate the tamper detection device and to facilitate logging of tamper and access events on the programmable device on the canister. In a preferred embodiment, de-activation of the tamper-prevention means occurs only when activated by a signal (or power) from the dispensing/filling apparatus.

Referring now to Figure 8, a filling meter is shown for automated filling of medication into the dosage compartments of a canister attached to the apparatus. The filling meter may be connected to the apparatus by physical means such as a cable or connector, or wirelessly, for automated filling in accordance with a patient's prescription or other dosage information. Alternatively, the filling meter may be a permanent component of the apparatus as may be the case for an apparatus located at a pharmacy. It is to be understood, however, the at the filling meter is not compulsory, and an operator can fill the compartments manually using dosage information provided through the dispensing system. The filling meter may be mounted on the apparatus in various appropriate positions identified, for example, at 603.

Preferably, the apparatus has a screen 604 for displaying information relating to a patient's dosage regimen. The screen may also display information and report data relevant to the patient from a system external to the apparatus, where a connection to an external system is provided. Such information may originate from a pharmacy, care institution, physician or other source and may include real time (or delayed) updates of information relating to the medication being dispensed. Remote publishing of this kind can -alert a pharmacist or patient to new information including medication contraindications or additional health benefits associated with the medication being filled into or dispensed from the patient's canister(s).

Information can be transmitted from the filling meter to the programmable device on the canister during or after filling of dosage compartments. This ensures that information relating to the patient, the patient's medication and the dosage regimen is retained on the canister enabling the canister transfer the information to other apparatus. This information can also be displayed on a display screen 604 of the apparatus and/or uploaded to another system via a communication network.

The apparatus includes drive shaft 605 having a shaped tip 607 that fits into to the locking slot in the drive clamp of the canister. The apparatus may verify that the shaped tip is locked in position by a rotational push. In order to rotate the dispensing wheel within the canister, the apparatus extends the end of the drive shaft 605, by any suitable means, for example, hydraulically or using extra threaded extension, with sufficient force to overcome the strength of the spring fitted on the axle thereby disengaging the teeth on the dispensing wheel from the teeth on the canister shell.

Drive shaft 605 rotates to the appropriate position for the dosage to be dispensed, under control of the control means. The drive shaft 605 may be complemented with a vibrating mechanism to agitate the canister to aid egress of medication from a dosage compartment. Where medication becomes jammed in the canister, the apparatus may have a special tool which fits into the dosage compartment while the canister is fitted to the apparatus to free up the jammed dosage.

Lug holders 606 include means to release the locking spring pins fitted to the canister. Once these pins are released and the teeth on the dispensing wheel are disengaged from the teeth on the canister shell, the dispensing wheel can be rotated to align a dosage compartment with the canister shell opening. The apparatus may also have a shelf 608 having a shaped depression for holding dosage cups for the dispensed dosage(s) to fall into, ready for consumption by the patient. Shelf 608 may also vibrate to separate medication such as capsules, tablets etc when one dispensed tablet or capsule is arranged on -top of or partially obscures -another when released from the canister. This enables identification by an optical detection device which may be incorporated into the apparatus and used to verify that a dosage has been accurately dispensed.

The apparatus also includes various communication ports 609. These ports may include but are not limited to USB, firewire, serial and parallel ports, or other connectors for adjusting or upgrading functionality of the system. The apparatus may further include communication slots for external computers, personal computing devices such as digital assistants, wireless enabling technology, phones, broadband cable, modems, bluetooth devices and the like. These do not form an exhaustive list as would be appreciated by the skilled addressee.

During use of the system, once a dosage has been dispensed the canister is rotated in the opposite direction to disengage the lugs and lug holders 606. The canister may also be engaged in a second configuration, with the delivery space at the top, without the interlocking of the gate, which is depressed on its hinge in that position.

In one embodiment, the programmable device(s) on the canister is configured to interact through the apparatus with a database. The database may also contain information regarding contraindications for certain drug-drug and drug-lifestyle contraindications to which the user may be alerted when medication is dispensed. Preferably, the canister also includes a tracking device such as a radio frequency transmitter or global positioning system to locate the whereabouts of canisters which may be misplaced by patients who suffer from dementia, Alzheimer's disease or other mental illness.

Filling and dispensing apparatus according to the present invention are usually used on a bench, tabletop or other support surface, although the apparatus may also be wall mounted, for example. Whilst the embodiment illustrated in Figure 6 shows the filling/dispensing apparatus "lying on its side", the apparatus may, in another position, be considered to be "lying on its back" (not illustrated). In the latter position, the gate and drive shaft face upward and the canister is attachable to the apparatus in a horizontal orientation rather than a vertical one.

The faceplate of the canister can then be removed (using the requisite keys and/or security -codes)- to expose the wheel with all- its compartments without any contents falling out. This permits removal of the outer rim(s) of the wheel for cleaning and/or replacement. In this configuration, wheel compartments may also be filled manually or automatically by use of a filling meter in a procedure similar to that described above. More than one filling meter may be used when the apparatus is in the second configuration, thereby increasing the speed with which the dosage compartments can be filled. In this orientation it may also be possible to fill more than one outer rim one at a time.

Preferably, the apparatus is configured for communication with a pharmacy or other remotely located information system in order to receive dosage and filling information and any other relevant information regarding the patient's medication. The information flow is secure and encrypted where necessary to maintain patient confidentiality, whist providing an audit trail to track medication dispensed from and filled into canisters. Most medication containers have barcodes or other labelling to identify the container contents, and, in a preferred embodiment, the filling apparatus is configured to scanned such labels to verify and automate medication being filled into a canister. Alternatively, RFIDs or other packaging technology may be used.

To dispense a medication dosage from a canister attached to the apparatus, an identification process takes place to ensure that the patient (or carer) is the correct recipient of the medication. When the necessary verification is complete, the apparatus drives the dispensing wheel to align the correct dosage compartment with the canister shell opening for medication delivery in accordance with dispensing rules governing dosage amounts, time of day, date and any other factors relevant to the patient's medication regimen. All or part of this information is stored on the programmable device(s) on the canister.

Preferably, dispensed dosages are communicated, either in real time or via delayed, periodic batch data transfer, to a remote database to track medication dispensing and usage. This provides an audit trail of medication transactions which enables pharmacies, hospitals and other care institutions to keep records for research and analysis, and to bill an entity such as a government agency, Medicare, hospital, health insurer or regulatory body.

Additionally, the apparatus may be configured to communicate with an information system of a facility through which the patient is treated. This communication enables the patient to receive treatment updates, and also enables the facility to monitor the patient's medication adherence. The facility can also alert and/or update the apparatus and particular canisters which may be attached to the apparatus with relevant and timely information relating to the patient's medication and dosage regimen and adjustments to the patient's prescription, for example. The information flow in the care facility also provides audit trails for dosage regimens, personnel involved and other relevant information for more effective and efficient management of patients and their medication. It also permits remote observation of cases and review of treatment guidelines based on changes in a monitored patient's condition.

Two way communication may also be provided via the apparatus for patients in their own homes. The levels of communication and services-provided (including information updates, billing, monitoring and alerts etc.) may be selected based upon a prearranged service agreement, and the patient charged accordingly. All information is secure and guarantees patient confidentiality.

The apparatus may also have an alert system. This alert system may be used in any environment but is envisaged to be of greatest value in a patient's own home. The alert system may be used to provide a reminder alarm to remind a patient that a dosage is due or overdue to be taken. It may also escalate alarms to the patient and/or carers to ensure correct dosages are dispensed to the patient and provide early notification of any dosage irregularity which can be transmitted to a monitoring service or other designated party. Designated parties may include a family member, carer, physician or emergency department, depending on the alarm escalation level.

Preferably, the apparatus can communicate with the patient or other designated contact by SMS, mobile phone or landline voice message, email, facsimile, pager or other communication medium. This communication can be used to remind the patient to take dosages that have been dispensed by the apparatus. The apparatus may also provide an emergency alarm system operable by the patient to indicate that the patient has fallen or requires assistance, where the apparatus relays this information to a carer or emergency organisation for action.

The inventive system dispenses correct dosages, at correct times, on the correct days securely. Advantageously, access to medication within the canister is restricted since access cannot be gained unless the canister is attached to a dispensing apparatus, and rules for dispensing (including information stored on the programmable device on the canister) have been satisfied. Moreover, embodiments of the invention provide a tamper-proof canister which also prevents unauthorised manual access to medication in the canister. The system also has the capability to receive and use new information, such as medication information and prescription adjustments, appropriately. It may also be configured to dispense dosages in advance so that patients can take their medication away from the apparatus and on outings whilst adhering to their dosage regime. These advance dosages can be limited to a predetermined maximum quantity to ensure safety. The advance dosage(s) dispensed can be loaded into a specially designed container (not shown) with separate compartments labelled am (morning), noon (lunch), pm (evening) or the like.

The apparatus is preferably powered by mains electricity, but may also have a battery and/or backup power supply. The apparatus may be portable and able to be simply re-connected with no loss of functionality or memory.

Figure 8 illustrates a simplified filling meter for attachment to a filling apparatus according to an embodiment of the present invention. The filling meter interacts with the apparatus through a cable 801. This cable may also provide power to the filling meter via the apparatus. Alternatively, the filling meter may communicate with the apparatus wirelessly, and have its own power supply. The filling meter measures dosages filled into individual dosage compartments of the dispensing wheel and can communicate with the filling apparatus (and hence the canister being filled) to maintain a record of dosages loaded into the canister. The record is thus transferable, on the canister, to any apparatus to which the canister is attached. The dosages are preferably filled automatically using an automatic filling mechanism that distinguishes different drugs from each other or manually using a counter 803.

A hopper 804 enables larger numbers of pills, capsules and the like to be loaded. It can form part of a dosage sorting system, which can interact with or be separate from the electronic technology of the filling meter. Shaped spout 805 is preferably removable and replaceable with a different shaped spout suited to the different dosage compartment configurations which may be fitted to the canister being filled. Preferably, the spout fits- snugly inside the canister shell opening to minimise medication spillage.

A measuring means or counter 803 can be used to determine the quantity of medication being introduced into each dosage compartment. The counter 803 is preferably electronic and may employ an infra red or "electronic eye" or other suitable technology to identify and count units of medication. The counter may include image processing software. A scale to weigh the medication may also be included with the filling meter or used as an alternative to the electronic identification devices. Preferably, the counter is configured to determine the size of a medication unit (pill, capsule or the like) as it passes by, as well as count the number of units. This involves three dimensional spatial recognition algorithms.

The filling meter may also be used to provide input to audit trails. Used in concert with the identification of drug types using RFID and bar coding as well as smart card and other technology for personnel identification, the filling meter can provide a link in the medication dispensing system and may also be used to modify drug handling procedures in pharmacies. The current practice of individual patients having individual boxes/containers of drugs may be shifted to employ more of a "bulk handling" approach, as the number of drugs dispensed for an individual patient is traceable and auditable.

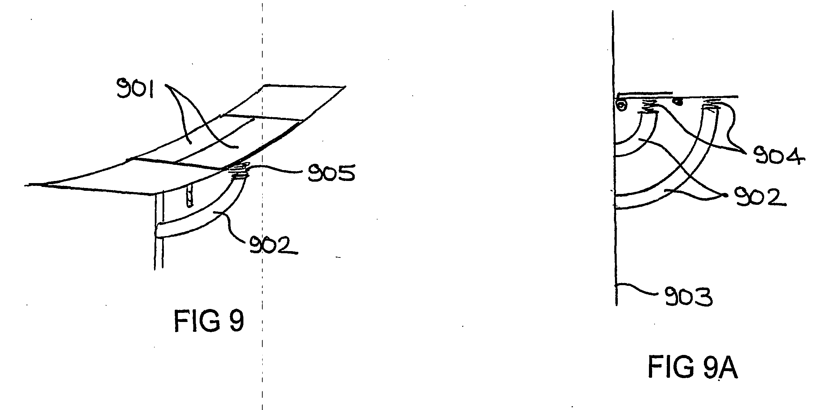

Figure 9 illustrates a pair of hinged gates 901 on a dispensing and/or filling apparatus. The gate portions 901 may be operated as a single unitary gate or individually operated as separate gates. When operated as a pair, gates 901 permit egress of medication from the entire delivery space opening in the canister shell. Alternatively gate portions 901 can be operated independently of each other to deliver dosages by partial opening of the delivery space. This is suitable for a canister having, for example, two outer rims fitted, where adjacent dosages in those rims are to be delivered independently of each other. Hinges 902 extending from the body of the apparatus each have a spring 904 at the gate end to allow the gate to be depressed slightly, for snug fitting into the delivery space of the canister. The gate(s) may have more than two hinged parts.

The hinge and spring arrangement is controlled by the control means (not shown) according to dosage information stored on the canister and permits synchronized and timely drug release, loading and removal of the canisters. The edges of the gate components are shaped to allow the canister to rotate smoothly into position and be rotated smoothly in the opposite direction for removal from the apparatus.

Using the present invention, existing practices where individual patients have individual boxes/containers of drugs obtained via separate prescriptions can be replaced with a "bulk handling" approach. Using this approach, numerous prescribed medications can be combined and allocated into dosage compartments of a canister without requiring the patient (or carer) to obtain separately, medication according to each individual prescription. This is particularly useful for a patient being treated for diabetes, high blood pressure and high cholesterol, as an example. These medications would all be filled into a single canister by the pharmacy in accordance with the patient's prescriptions and released from the canister dose by dose according to instructions from the prescribing physician(s), using a dispensing apparatus.

Complex combinations of dosage regimes are accommodated by the flexible canister rim configurations, and drug wastage may be eliminated as unused medications may be recycled back into circulation if not dispensed to a patient within a particular time frame. This is particularly important given the amount of prescription medication which is currently disposed of in the rubbish, and not returned to a pharmacy. Moreover, because of the security of the canisters, the inventive system can provide a pharmacy-based secure medication distribution system in which canisters filled with prescribed drugs can be sent from a pharmacy to a patient by regular post or using a courier.

Alerts, reminders and provision of patient, drug and other information using the inventive dispensing system add premium features which provide additional advantages not available with existing medication dispensing systems. Moreover, since drugs filled into canisters and dispensed to individual patients are traceable and auditable, the inventive system provides economic and sociologic advantages not provided by the prior art.

It is to be understood that the embodiments illustrated and described herein are examples only. Various alterations, modifications and additions may be made to the parts previously without departing from the scope or ambit of the invention.

Applications of information flow in the present invention will now be more fully described with reference to the following non-limiting examples.

### Examples

### Example 1 - In the pharmacy

- The filling apparatus reads the patient information from the canister.
- The patient's record is accessed on the pharmacy information system by the control means.
- The system displays tamper detection status information for the pharmacy staff. Depending on the tamper detection status, the canister may be referred to a workshop for tamper-proof testing and repaired/replaced and recoded if necessary.
- The system displays dispensing information for the pharmacy staff.
- The dispensing information displays the dosages and their time of day and date.
- The system allocates dosage compartments to each required dosage.
- The drug ID is scanned to the system.
- The drugs are delivered through the filling meter.
- The meter passes relevant information to the filling apparatus and the wheel is rotated to the correct dosage compartment for each medication dosage in the regimen for that canister.
- The system acknowledges canister filling is complete. Relevant information including drug, time, dispensing personnel identification etc is recorded on the canister and on a central database for further processing and tracking.
- The canister is then scanned at dispatch for time, destination and courier etc information.
- Prescription alterations in the nursing home environment can be automatically sent to the pharmacy so that incoming canisters are automatically updated before they are re-filled.
- Doctors can send prescription updates to pharmacies (or pharmacies can interrogate a central database) so that canisters can be automatically updated with changes to a prescription.
- Pharmacists and doctors can interrupt or alter a dosage cycle based upon new information

### Example 2 - In the nursing home

- Canisters are received, scanned and recorded when they arrive and information is sent to the relevant patient's information record.
- Canisters are distributed to patients in the facility; the person in charge of dispensing drugs enters an ID at sign-on.
- Each canister identifies itself on the Nursing home dispensing apparatus as belonging to a particular patient.
- The dispensing apparatus reads the relevant information from the canister the canister is moved into position for dispensing.
- The system displays tamper detection status information for the nursing staff. Depending on the tamper detection status, the canister may be referred to a workshop for tamper-proof testing and repaired/replaced and recoded if necessary.
- The dosages to be dispensed are displayed on the apparatus's screen and/or the screen of a PDA carried by the person in charge and/or a laptop computer moving with the person in charge.
- The patient's ID card, smart card or bar-coded wrist band, or that of the person in charge is read and verified by the dispensing apparatus and the dosage dispensed. The facility's patient information system is updated accordingly.
- If patient's ID is not entered, or the person in charge aborts the dispensing; the dosage is not dispensed. The facility's patient information system is updated accordingly.
- The dispensing apparatus completes dispensing the dosage and the person in charge re-enters ID for sign-off.
- The technology on the canister determines if a patient is due for medication from the filling regimen and this information is available to the nursing system.
- The nursing home patient information system is updated throughout the dosage regimen and reordering of canisters is facilitated.
- Prescription alterations identified in the nursing home can be automatically transmitted to the pharmacy so that dosages filled into incoming canisters are automatically corrected.
- Doctors can send prescription updates to pharmacies so that canisters can be automatically updated.
- The regimen for Group homes is similar to that of nursing homes and will be modified where needed.

### Example 3 - In the patient's home

- Home dispensing apparatus connected to monitoring service (this service is preferably provided over a telephone line and does not require connection of a dedicated computer system)
- A canister from a pharmacy is loaded on the apparatus and dosage regimen information is accepted by the dispensing apparatus.
- The system displays tamper detection status information remotely for the pharmacy staff. Depending on the tamper detection status, the canister may be referred to a workshop for tamper-proof testing and repaired/replaced and recoded if necessary.
- Dispensing apparatus moves dispensing wheel to correct dosage position.
- Dispensing apparatus alerts patient that medication is due to be taken.
- Patient enters ID, then dosage is dispensed, record updated and held by apparatus.
- The dosage update information is held by the dispensing apparatus for a preset period then uploaded to the service by phone, e.g. daily clearing of information.
- If the patient does not enter ID within a certain time frame after alert the system then commences alarm escalation procedures that have been preset depending upon the patient's medical status, mental capacity and need for medication(s).
- The escalation procedures include phone call from monitoring service to patient, phone call from service to patient's designated carer and then to emergency service provider.
- The monitoring service provides a medication information update service that uses expert system data mining to provide real time updates on medication and other relevant information to the client. This service can be provided to nursing homes and other care facilities also.
- The information from the service can be printed out on a printer connected to the system.
- Doctors can send information to the patient through the service.

## Claims

1. A medication dispensing system including:
a filling apparatus;
a dispensing apparatus;
a portable, refillable canister having a plurality of dosage compartments,
the canister being releasably mountable on the filling apparatus and dispensing apparatus and having one or more programmable devices configured to communicate with the filling and dispensing apparatus; and
control means configured to control filling of medication into and egress of medication out of a canister attached to a filling/dispensing apparatus in accordance with information stored by the one or more programmable devices on the canister;
**characterised in that** the filling apparatus is configured to fill the dosage compartments automatically with medication in accordance with a patient's prescription communicated to the filling apparatus.

2. A medication dispensing system according to claim 1 wherein the canister includes a programmable device for each dosage compartment.

3. A medication dispensing system according to claim 1 or claim 2 wherein the one or more programmable devices are configured to store information on the canister including one or more of:
a canister identity;
configuration of dosage compartments in the canister;
number of dosage compartments in the canister;
a medication or plurality of medications contained in a dosage compartment;
a time at which a dosage was dispensed;
a time at which a dosage is due to be dispensed; and
an identity of a patient to whom the canister is allocated.

4. A medication dispensing system according to any one of the preceding claims wherein programmable devices on the canister include one or more of:
a radio frequency identification device (RFID);
a microchip;
a smart card;
a subscriber identity module (SIM) chip;
a barcode; and
a magnetic strip.

5. A medication dispensing system according to any one of the preceding claims further including tamper-prevention means on the canister to prevent unauthorised access to medication within the canister.

6. A medication dispensing system according to claim 5 wherein the canister further includes tamper-detection means for detecting tamper attempts.

7. A medication dispensing system according to claim 5 or claim 6 wherein the tamper-prevention means includes a device operable only by application of a signal from the dispensing/filling apparatus.

8. A medication dispensing system according to any one of claims 5 to 7 wherein a programmable device on the canister stores information relating to a tamper attempt.

9. A medication dispensing system according to claim 8 wherein programmable device on the canister further includes a tamper log to record changes in the tamper-detection status.

10. A medication dispensing system according to any one of claims 5 to 9 wherein a programmable device on the canister is configured to communicate detection of a tamper attempt to a dispensing/filling apparatus or to a remote service.

11. A medication dispensing system according to any one of the preceding claims wherein the canister further includes a tracking device to track the canister's location.

12. A medication dispensing system according to any one of the preceding claims, wherein the filling apparatus and the dispensing apparatus are incorporated into a single unit.

13. A medication dispensing system according to any one of the preceding claims, wherein the canister includes:
a canister shell; and
a dispensing wheel having a plurality of dosage compartments, the
dispensing wheel being rotatably couplable to the canister shell;
wherein rotation of the dispensing wheel relative to the canister shell is controlled by the control means to facilitate filling of medication into and egress of medication out of the dosage compartments through an opening in the canister shell.

14. A medication dispensing system according to any one of the preceding claims, wherein the dispensing apparatus includes:
one or more dispensing zones which are independently operable under control of the control means to facilitate filling of medication into or egress of medication out of the dosage compartments.

15. A medication dispensing system according to any one of the preceding claims, wherein one or more of the filling and dispensing apparatus further includes a device reader for reading the one or more programmable devices on one or more canisters.

16. A medication dispensing system according to any one of the preceding claims, wherein one or more of the filling apparatus and the dispensing apparatus is in communication with a remotely located information service.

17. A medication dispensing system according to claim 16, wherein the dispensing apparatus further includes a communication component for transferring information relating to dispensed medication to the remotely located information service.

18. A medication dispensing system according to any one of claims 1 to 17, wherein the patient's prescription is communicated to the filling apparatus from:
a canister;
a central database;
a remotely located physician;
a pharmacist;
a hospital; or
another entity authorized to dispense and/or prescribe medication.

19. A medication dispensing system according to claim 18 wherein the filling apparatus further includes a filling meter for measuring a quantity of medication units being filled into a dosage compartment of a canister.

20. A medication dispensing system according to claim 19, wherein the filling meter is in communication with the control means for automated filling of medication units into dosage compartments.

21. A medication dispensing system according to claim 19 or claim 20, wherein the filling meter includes one or more of:
a counter;
a scale;
electronic eye;
optical recognition device; and
a camera;
suitable for quantitating medication units being filled into the dosage compartment.

22. A medication dispensing system according to any one of the preceding claims further including a billing interface to bill automatically a patient, employer, healthcare provider, insurance company or other billable entity for medication dispensed from the system.

23. A medication dispensing system according to any one of the preceding claims wherein the filling apparatus further includes a printer configured to print a label affixable to the canister, the label displaying information relevant to a patient's dosage regimen.

24. A medication dispensing system according to claim 23 wherein the printer is configured to print automatically a new label containing new information when the patient's dosage regimen changes.

25. A medication dispensing system including:
a dispensing apparatus;
a portable, refillable canister having a plurality of dosage compartments,
the canister being releasably mountable on dispensing apparatus and having one or more programmable devices configured to communicate with the dispensing apparatus; and
control means configured to control the dispensing apparatus to facilitate egress of medication from a dosage compartment in accordance with information stored by the one or more programmable devices on the canister;
**characterised in that** the dosage compartments are filled automatically with medication in accordance with a patient's prescription and medication is dispensed from the dosage compartments in accordance with information stored by the one or more programmable devices on the canister, said information pertaining to the patient's pre-determined medication dosage regimen.

26. A medication dispensing system according to claim 25 wherein the canister includes a programmable device for each dosage compartment.

27. A medication dispensing system according to claim 25 or claim 26 wherein the canister further includes tamper-prevention means to prevent unauthorised access to medication within the canister.

28. A medication dispensing system according to claim 27 wherein the canister further includes tamper-detection means for detecting a tamper attempt.

29. A medication dispensing system according to claim 27 or claim 28 wherein tamper-prevention means includes a device operable only by application of a signal from the dispensing/filling apparatus.

30. A medication dispensing system according to claim 28 wherein programmable device on the canister further includes a tamper log to record changes in the tamper-detection status.

31. A medication dispensing system according to any one of claims 28 to 30 wherein a programmable device on the canister is configured to communicate detection of a tamper attempt to a dispensing/filling apparatus and/or a remote service.

32. A medication dispensing system according to any one of claims 25 to 31 wherein the canister further includes a tracking device to track the canister's location.

33. A medication dispensing system according to claim any one of claims 25 to 32, wherein the canister further includes:
a canister shell; and
a dispensing wheel having a plurality of dosage compartments, the
dispensing wheel being rotatably couplable to the canister shell;
wherein rotation of the dispensing wheel relative to the canister shell is controlled by the control means to facilitate egress of medication from a dosage compartment through an opening in the canister shell.

34. A medication dispensing system according to any one of claims 25 to 33, wherein the dispensing apparatus includes:
one or more dispensing zones which are independently operable under control of the control means to facilitate egress of medication from the dosage compartments.

35. A medication dispensing system according to any one of claims 25 to 34 wherein a programmable identification device includes one or more of:
a radio frequency identification device (RFID);
a microchip;
a subscriber identity module (SIM) chip;
a smart card;
a barcode; and
a magnetic strip.

36. A medication dispensing system according to any one of claims 25 to 35, wherein the dispensing apparatus further includes a device reader for reading the one or more programmable devices on the canister.

37. A medication dispensing system according to any one of claims 25 to 36, further including a billing interface to bill automatically a patient, employer, healthcare provider, insurance company or other billable entity for medication dispensed from the system.

38. A medication dispensing system according to any one of claims 1 to 35, wherein the dispensing apparatus further includes a display means to visually indicate information including one or more of time of day, time next dosage is due to be dispensed, identity of patient, identity of medication, contact details in case of emergency, and dosage information.

39. A medication dispensing system according to any one of the preceding claims configured to store electronically a record of canister-access events including but not limited to an identifier for a person accessing the canister, a time at which the canister was accessed and the location of the access.

40. A medication dispensing system according to any one of the preceding claims further including a movable member for separating dispensed medication units so they can be identified by an optical detection device.

41. A medication dispensing system according to any one of the preceding claims wherein the dispensing apparatus further includes an alarm system for alerting a patient that a dosage of medication is due to be dispensed.

42. A medication dispensing system according to claim 41, wherein the alarm system raises an alert when a patient has not dispensed a prescribed medication in a pre-determined time frame.

43. A medication dispensing system according to claim 42, wherein there is a plurality of predetermined time frames and corresponding alert levels, and wherein elapsing of a longer time frame escalates the alert level to a higher alert level.

44. A medication dispensing system according to claim 41, wherein the alarm system automatically executes an alert procedure according to an alert level, the alert procedure includes one or more of:
sounding a warning sound;
transmitting a message to a communication device of the patient;
transmitting a message to a carer of the patient;
transmitting a message to the patient's healthcare facility;
transmitting a message to the patient's physician; and
transmitting an alert to an emergency organisation.

45. A medication dispensing system according to any one of claims 41 to 44, wherein the alarm system can be used by a patient to send a distress alert to one or more of:
the control means;
a carer of the patient;
a healthcare facility; and
a physician.

46. A medication dispensing system according to any one of the preceding claims, wherein the control means controls egress of medication to a patient in accordance with one or more pre-determined dispensing rules stored by a programmable device on the canister, the rules including one or more of:
has the patient been correctly identified?
has the canister been correctly identified?
is the medication to be dispensed correct?
is the time for dispensing medication correct?
is the canister free of tamper attempts? and
is the medication absent of contraindication with other prescribed medication?

47. A medication dispensing system according to any one of the preceding claims, wherein the control means automatically updates dosage information stored by the programmable device on the canister.

48. A canister for use with a medication dispensing system, the canister including:
a canister shell having a dispensing opening;
a dispensing wheel having a plurality of dosage compartments and being rotatably couplable to the canister shell; and
one or more programmable devices configured to communicate with apparatus for filling or dispensing medication;
wherein medication is filled into or dispensed from the canister by attaching it to a filling and/or dispensing apparatus having a control means, and wherein the control means controls rotation of the dispensing wheel relative to the canister shell to facilitate filling of medication into and egress of medication out of the dosage compartments in accordance with the information stored on the one or more programmable devices on the canister;
**characterised in that** the dosage compartments are filled automatically with medication in accordance with a patient's description and the information stored on the one or more programmable devices on the canister pertains to a patient's pre-determined medication dosage regimen.

49. A canister according to claim 48, further including a programmable device for each dosage compartment in the dispensing wheel.

50. A canister according to claim 48 or claim 49, wherein the one or more programmable devices store information including one or more of canister identity, configuration of dosage compartments in the canister, number of outer rim sections in the dispensing wheel, number of dosage compartments in the canister, a medication or plurality of medications contained in a dosage compartment, a time at which a dosage was dispensed or is due to be dispensed, and an identity of a patient to whom the canister is allocated.

51. A canister according to claim 49 wherein a programmable device on the canister includes one or more of:
a radio frequency identification device (RFID);
a microchip;
a subscriber identity module (SIM) chip;
a smart card;
a barcode; and
a magnetic strip.

52. A canister according to any one of claims 48 to 51 further including tamper prevention means for preventing access to the canister when the filling or dispensing rules have not been satisfied.

53. A canister according to any one of claims 48 to 52 wherein the canister further includes tamper-detection means for detecting tamper attempts.

54. A canister according to claim 53 wherein programmable device on the canister maintains a tamper log to keep a record of tamper attempts.

55. A canister according to claim 52 wherein the tamper prevention means includes a device operable only by application of a signal from the dispensing/filling apparatus.

56. A canister according to claim 53 or claim 54 wherein the canister is configured to communicate detection of a tamper attempt to a central administrator or other authorized entity.

57. A canister according to any one of claims 48 to 56 wherein the dispensing wheel is adapted to receive one or more interchangeable rim portions having various dosage compartment configurations.

58. A method of dispensing medication to a patient using a medication dispensing system according to any one of claims 1 to 47.

## Patentansprüche

1. Ein Medikationsausgabesystem umfassend:
einen Füllapparat;
einen Ausgabeapparat;
einen tragbaren, nachfüllbaren Kanister mit einer Vielzahl von Dosierungskompartimenten, wobei der Kanister lösbar auf dem Füllapparat und Ausgabeapparat angebracht werden kann und einen oder mehr programmierbare Vorrichtungen hat, die für die Kommunikation mit dem Füll- und Ausgabeapparat konfiguriert sind; und
Kontrollmittel konfiguriert für die Kontrolle des Einfüllens von Medikation in und Austritts von Medikation aus einem an einem Füll-/Ausgabeapparat befestigten Kanister in Übereinstimmung mit Information, die durch die ein oder mehr programmierbaren Vorrichtungen auf dem Kanister gespeichert ist;
**gekennzeichnet dadurch, dass** der Füllapparat konfiguriert ist für die automatische Befüllung der Dosierungskompartimente mit Medikation in Übereinstimmung mit dem dem Füllapparat mitgeteilten Rezept eines Patienten.

2. Ein Medikationsausgabesystem gemäß Anspruch 1, wobei der Kanister eine programmierbare Vorrichtung für jedes Dosierungskompartiment umfasst.

3. Ein Medikationsausgabesystem gemäß Anspruch 1 oder Anspruch 2, wobei die ein oder mehr programmierbaren Vorrichtungen konfiguriert sind für die Speicherung von Information auf dem Kanister umfassend eines oder mehr von:
einer Kanisteridentität;
Konfiguration der Dosierungskompartimente im Kanister;
Anzahl der Dosierungskompartimente im Kanister;
einer Medikation oder Vielzahl von Medikationen, die in einem Dosierungskompartiment enthalten ist;
einer Zeit, zu der eine Dosis ausgegeben wurde;
einer Zeit, zu der eine Dosis ausgegeben werden soll; und
einer Identität eines Patienten, dem der Kanister zugeordnet ist.

4. Ein Medikationsausgabesystem gemäß irgendeinem der vorherigen Ansprüche, wobei programmierbare Vorrichtungen auf dem Kanister eines oder mehr von:
einer Radiofrequenzerkennungsvorrichtung (RFID);
einem Mikrochip;
einer Smartcard;
einem Subscriber-Identity-Module-(SIM)-Chip;
einem Barcode; und
einem Magnetstreifen
umfassen.

5. Ein Medikationsausgabesystem gemäß irgendeinem der vorherigen Ansprüche weiter umfassend Manipulationsvorbeugungsmittel auf dem Kanister, um unbefugten Zugang zu Medikation innerhalb des Kanisters zu verhindern.

6. Ein Medikationsausgabesystem gemäß Anspruch 5, wobei der Kanister weiter Manipulationsentdeckungsmittel zum Nachweis von Manipulationsversuchen umfasst.

7. Ein Medikationsausgabesystem gemäß Anspruch 5 oder Anspruch 6, wobei das Manipulationsvorbeugungsmittel eine Vorrichtung umfasst, die nur durch Einsatz eines Signals vom Ausgabe-/Füllapparat betätigt werden kann.

8. Ein Medikationsausgabesystem gemäß irgendeinem der Ansprüche 5 bis 7, wobei eine programmierbare Vorrichtung auf dem Kanister Information betreffend einen Manipulationsversuch speichert.

9. Ein Medikationsausgabesystem gemäß Anspruch 8, wobei programmierbare Vorrichtung auf dem Kanister weiter umfasst ein Manipulationslog zur Aufzeichnung von Veränderungen im Manipulationsentdeckungsstatus.

10. Ein Medikationsausgabesystem gemäß irgendeinem der Ansprüche 5 bis 9, wobei eine programmierbare Vorrichtung auf dem Kanister konfiguriert ist für die Mitteilung der Entdeckung eines Manipulationsversuchs an einen Ausgabe-/Füllapparat oder an einen Ferndienst.

11. Ein Medikationsausgabesystem gemäß irgendeinem der vorherigen Ansprüche, wobei der Kanister weiter umfasst eine Ortungsvorrichtung zur Verfolgung der Position des Kanisters.

12. Ein Medikationsausgabesystem gemäß irgendeinem der vorherigen Ansprüche, wobei der Füllapparat und der Ausgabeapparat in eine einzige Einheit eingegliedert sind.

13. Ein Medikationsausgabesystem gemäß irgendeinem der vorherigen Ansprüche, wobei der Kanister:
eine Knisterhülle; und
ein Ausgaberad mit einer Vielzahl von Dosierungskompartimenten umfasst, wobei das Ausgaberad drehbar an die Kanisterhülle gekoppelt werden kann;
wobei die Drehung des Ausgaberades relativ zur Kanisterhülle durch das Kontrollmittel kontrolliert wird, um das Einfüllen von Medikation in und den Austritt von Medikation aus den Dosierungskompartimenten durch eine Öffnung in der Kanisterhülle zu erleichtern.

14. Ein Medikationsausgabesystem gemäß irgendeinem der vorherigen Ansprüche, wobei der Ausgabeapparat:
ein oder mehr Ausgabezonen umfasst, die unter Kontrolle des Kontrollmittels unabhängig betätigt werden können, um das Einfüllen von Medikation in und den Austritt von Medikation aus den Dosierungskompartimenten zu erleichtern.

15. Ein Medikationsausgabesystem gemäß irgendeinem der vorherigen Ansprüche, wobei eines oder mehr des Füll- und Ausgabeapparats weiter umfasst einen Vorrichtungsleser zum Ablesen der ein oder mehr programmierbaren Vorrichtungen auf einem oder mehr Kanistern.

16. Ein Medikationsausgabesystem gemäß irgendeinem der vorherigen Ansprüche, wobei eines oder mehr von dem Füllapparat und dem Ausgabeapparat in Kommunikation mit einem entfernt befindlichen Informationsdienst steht.

17. Ein Medikationsausgabesystem gemäß Anspruch 16, wobei der Ausgabeapparat weiter umfasst eine Kommunikationskomponente zur Übertragung von Information betreffend ausgegebener Medikation an den entfernt befindlichen Informationsdienst.

18. Ein Medikationsausgabesystem gemäß irgendeinem der Ansprüche 1 bis 17, wobei das Rezept des Patienten dem Füllapparat mitgeteilt wird von:
einem Kanister;
einer zentralen Datenbank;
einem entfernt befindlichen Arzt;
einem Pharmazeuten;
einem Krankenhaus; oder
einer anderen zur Ausgabe und/oder Verschreibung von Medikation befugten Einheit.

19. Ein Medikationsausgabesystem gemäß Anspruch 18, wobei der Füllapparat weiter umfasst einen Füllmesser zur Messung der Menge an Medikationseinheiten, die in ein Dosierungskompartiment eines Kanisters gefüllt werden.

20. Ein Medikationsausgabesystem gemäß Anspruch 19, wobei der Füllmesser zum automatisierten Abfüllen von Medikationseinheiten in Dosierungskompartimente in Kommunikation mit dem Kontrollmittel steht.

21. Ein Medikationsausgabesystem gemäß Anspruch 19 oder Anspruch 20, wobei der Füllmesser eines oder mehr von:
einem Zähler;
einer Waage;
elektronischem Auge;
optischer Erkennungsvorrichtung; und
einer Kamera;
geeignet zur Quantifizierung von Medikationseinheiten, die ins Dosierungskompartiment gefüllt werden, umfasst.

22. Ein Medikationsausgabesystem gemäß irgendeinem der vorherigen Ansprüche weiter umfassend eine Fakturierungsschnittstelle zur automatischen Belastung eines Patienten, eines Arbeitgebers, eines Gesundheitsdienstleisters, einer Versicherungsgesellschaft oder einer anderen belastbaren Einheit für aus dem System ausgegebene Medikation.

23. Ein Medikationsausgabesystem gemäß irgendeinem der vorherigen Ansprüche, wobei der Füllapparat weiter umfasst einen Drucker konfiguriert zum Drucken eines am Kanister befestigbaren Etiketts, wobei das Etikett Information anzeigt, die für das Dosierungsschema eines Patienten relevant ist.

24. Ein Medikationsausgabesystem gemäß Anspruch 23, wobei der Drucker konfiguriert ist, automatisch ein neues Etikett enthaltend neue Information zu drucken, wenn sich das Dosierungsschema des Patienten ändert.

25. Ein Medikationsausgabesystem umfassend:
einen Ausgabeapparat;
einen tragbaren, nachfüllbaren Kanister mit einer Vielzahl von Dosierungskompartimenten, wobei der Kanister lösbar auf Ausgabeapparat angebracht werden kann und einen oder mehr programmierbare Vorrichtungen hat,
die für die Kommunikation mit dem Ausgabeapparat konfiguriert sind; und
Kontrollmittel konfiguriert für die Kontrolle des Ausgabeapparats, um den Austritt von Medikation aus einem Dosierungskompartiment zu erleichtern in Übereinstimmung mit Information, die durch die ein oder mehr programmierbaren Vorrichtungen auf dem Kanister gespeichert ist;
**gekennzeichnet dadurch, dass** die Dosierungskompartimente in Übereinstimmung mit dem Rezept eines Patienten automatisch befüllt werden und Medikation aus den Dosierungskompartimenten ausgegeben wird in Übereinstimmung mit Information, die durch die ein oder mehr programmierbaren Vorrichtungen auf dem Kanister gespeichert ist, wobei sich diese Information auf das vorher bestimmte Medikationsdosierungsschema des Patienten bezieht.

26. Ein Medikationsausgabesystem gemäß Anspruch 25, wobei der Kanister eine programmierbare Vorrichtung für jedes Dosierungskompartiment umfasst.

27. Ein Medikationsausgabesystem gemäß Anspruch 25 oder Anspruch 26, wobei der Kanister weiter Manipulationsvorbeugungsmittel umfasst, um unbefugten Zugang zu Medikation innerhalb des Kanisters zu verhindern.

28. Ein Medikationsausgabesystem gemäß Anspruch 27, wobei der Kanister weiter Manipulationsentdeckungsmittel zum Nachweis von einem Manipulationsversuch umfasst.

29. Ein Medikationsausgabesystem gemäß Anspruch 27 oder Anspruch 28, wobei Manipulationsvorbeugungsmittel eine Vorrichtung umfasst, die nur durch Einsatz eines Signals vom Ausgabe-/Füllapparat betätigt werden kann.

30. Ein Medikationsausgabesystem gemäß Anspruch 28, wobei programmierbare Vorrichtung auf dem Kanister weiter umfasst ein Manipulationslog zur Aufzeichnung von Veränderungen im Manipulationsentdeckungsstatus.

31. Ein Medikationsausgabesystem gemäß irgendeinem der Ansprüche 28 bis 30, wobei eine programmierbare Vorrichtung auf dem Kanister konfiguriert ist für die Mitteilung der Entdeckung eines Manipulationsversuchs an einen Ausgabe-/Füllapparat und/oder an einen Ferndienst.

32. Ein Medikationsausgabesystem gemäß irgendeinem der Ansprüche 25 bis 31, wobei der Kanister weiter umfasst eine Ortungsvorrichtung zur Verfolgung der Position des Kanisters.

33. Ein Medikationsausgabesystem gemäß irgendeinem der Ansprüche 25 bis 32, wobei der Kanister weiter umfasst:
eine Kanisterhülle; und
ein Ausgaberad mit einer Vielzahl von Dosierungskompartimenten, wobei das Ausgaberad drehbar an die Kanisterhülle gekoppelt werden kann;
wobei die Drehung des Ausgaberades relativ zur Kanisterhülle durch das Kontrollmittel kontrolliert wird, um den Austritt von Medikation aus einem Dosierungskompartiment durch eine Öffnung in der Kanisterhülle zu erleichtern.

34. Ein Medikationsausgabesystem gemäß irgendeinem der Ansprüche 25 bis 33, wobei der Ausgabeapparat:
ein oder mehr Ausgabezonen umfasst, die unter Kontrolle des Kontrollmittels unabhängig betätigt werden können, um den Austritt von Medikation aus den Dosierungskompartimenten zu erleichtern.

35. Ein Medikationsausgabesystem gemäß irgendeinem der Ansprüche 25 bis 34, wobei eine programmierbare Erkennungsvorrichtung eines oder mehr von:
einer Radiofrequenzerkennungsvorrichtung (RFID);
einem Mikrochip;
einem Subscriber-Identity-Module-(SIM)-Chip;
einer Smartcard;
einem Barcode; und
einem Magnetstreifen
umfasst.

36. Ein Medikationsausgabesystem gemäß irgendeinem der Ansprüche 25 bis 35, wobei der Ausgabeapparat weiter umfasst einen Vorrichtungsleser zum Ablesen der ein oder mehr programmierbaren Vorrichtungen auf dem Kanister.

37. Ein Medikationsausgabesystem gemäß irgendeinem der Ansprüche 25 bis 36 weiter umfassend eine Fakturierungsschnittstelle zur automatischen Belastung eines Patienten, eines Arbeitgebers, eines Gesundheitsdienstleisters, einer Versicherungsgesellschaft oder einer anderen belastbaren Einheit für aus dem System ausgegebene Medikation.

38. Ein Medikationsausgabesystem gemäß irgendeinem der Ansprüche 1 bis 35, wobei der Ausgabeapparat weiter umfasst ein Anzeigemittel zur visuellen Anzeige von Information einschließlich eines oder mehr von Tageszeit, Zeit, zu der die nächste Dosis ausgegeben werden soll, Identität des Patienten, Identität der Medikation, Kontaktdetails im Notfall und Dosierungsinformation.

39. Ein Medikationsausgabesystem gemäß irgendeinem der vorherigen Ansprüche konfiguriert zur elektronischen Speicherung eines Verzeichnisses von Kanisterzugriffsereignissen einschließlich, aber nicht beschränkt auf, einer Kennung für eine Person, die auf den Kanister zugreift, einer Zeit, zu der auf den Kanister zugegriffen wurde, und der Position des Zugriffs.

40. Ein Medikationsausgabesystem gemäß irgendeinem der vorherigen Ansprüche weiter umfassend ein bewegliches Element zur Trennung von ausgegebenen Medikationseinheiten, so dass sie von einer optischen Erkennungsvorrichtung identifiziert werden können.

41. Ein Medikationsausgabesystem gemäß irgendeinem der vorherigen Ansprüche, wobei der Ausgabeapparat weiter umfasst ein Alarmsystem, um einen Patienten darauf hinzuweisen, dass eine Medikationsdosis ausgegeben werden soll.

42. Ein Medikationsausgabesystem gemäß Anspruch 41, wobei das Alarmsystem eine Warnung auslöst, wenn ein Patient eine verschriebene Medikation nicht in einem vorher bestimmten Zeitrahmen ausgegeben hat.

43. Ein Medikationsausgabesystem gemäß Anspruch 42, wobei es eine Vielzahl von vorher bestimmten Zeitrahmen und entsprechenden Warnstufen gibt, und wobei das Verstreichen eines längeren Zeitrahmens die Warnstufe zu einer höheren Warnstufe steigert.

44. Ein Medikationsausgabesystem gemäß Anspruch 41, wobei das Alarmsystem automatisch einen Warnvorgang gemäß einer Warnstufe durchführt, der Warnvorgang eines oder mehr von:
Ertönen eines Warngeräusches;
Übertragung einer Nachricht an eine Kommunikationsvorrichtung des Patienten;
Übertragung einer Nachricht an einen Pfleger des Patienten;
Übertragung einer Nachricht an die Gesundheitseinrichtung des Patienten;
Übertragung einer Nachricht an den Arzt des Patienten; und
Übertragung einer Warnung an eine Notfallorganisation
umfasst.

45. Ein Medikationsausgabesystem gemäß irgendeinem der Ansprüche 41 bis 44, wobei das Alarmsystem von einem Patienten verwendet werden kann, um eine Notfallwarnung an eines oder mehr von:
dem Kontrollmittel;
einem Pfleger des Patienten;
einer Gesundheitseinrichtung; und
einem Arzt
zu senden.

46. Ein Medikationsausgabesystem gemäß irgendeinem der vorherigen Ansprüche, wobei das Kontrollmittel den Austritt von Medikation an einen Patienten in Übereinstimmung mit einer oder mehr vorher bestimmten Ausgaberegeln kontrolliert, die durch eine programmierbare Vorrichtung auf dem Kanister gespeichert sind, wobei die Regeln ein oder mehr von:
ist der Patient korrekt identifiziert worden?
ist der Kanister korrekt identifiziert worden?
ist die auszugebende Medikation korrekt?
ist die Zeit zur Ausgabe der Medikation korrekt?
ist der Kanister frei von Manipulationsversuchen? und
ist die Medikation frei von Gegenanzeigen bezüglich anderer verschriebener Medikation?
umfassen.

47. Ein Medikationsausgabesystem gemäß irgendeinem der vorherigen Ansprüche, wobei das Kontrollmittel durch die programmierbare Vorrichtung auf dem Kanister gespeicherte Dosierungsinformation automatisch aktualisiert.

48. Ein Kanister zur Verwendung mit einem Medikationsausgabesystem, der Kanister umfassend:
eine Kanisterhülle mit einer Ausgabeöffnung;
ein Ausgaberad mit einer Vielzahl von Dosierungskompartimenten, das drehbar an die Kanisterhülle gekoppelt werden kann; und
ein oder mehr programmierbare Vorrichtungen konfiguriert für die Kommunikation mit dem Apparat zum Einfüllen oder Ausgeben von Medikation;
wobei Medikation eingefüllt wird in oder ausgegeben wird aus dem Kanister, indem er befestigt wird an einem Füll- und/oder Ausgabeapparat, der ein Kontrollmittel hat, und wobei das Kontrollmittel die Drehung des Ausgaberades relativ zur Kanisterhülle kontrolliert, um das Einfüllen von Medikation in und den Austritt von Medikation aus den Dosierungskompartimenten in Übereinstimmung mit der auf den ein oder mehr programmierbaren Vorrichtungen auf dem Kanister gespeicherten Information zu erleichtern;
**gekennzeichnet dadurch, dass** die Dosierungskompartimente automatisch mit Medikation befüllt werden in Übereinstimmung mit der Beschreibung eines Patienten und die auf der ein oder mehr programmierbaren Vorrichtungen auf dem Kanister gespeicherte Information sich auf das vorher bestimmte Dosierungsschema eines Patienten bezieht.

49. Ein Kanister gemäß Anspruch 48, weiter umfassend eine programmierbare Vorrichtung für jedes Dosierungskompartiment im Ausgaberad.

50. Ein Kanister gemäß Anspruch 48 oder Anspruch 49, wobei die ein oder mehr programmierbaren Vorrichtungen Information speichern einschließlich einem oder mehr von Kanisteridentität, Konfiguration der Dosierungskompartimente im Kanister, Anzahl der äußeren Randabschnitte im Ausgaberad, Anzahl der Dosierungskompartimente im Kanister, einer Medikation oder Vielzahl von Medikationen, die in einem Dosierungskompartiment enthalten ist, einer Zeit, zu der eine Dosis ausgegeben wurde oder ausgegeben werden soll, und einer Identität eines Patienten, dem der Kanister zugeordnet ist.

51. Ein Kanister gemäß Anspruch 49, wobei eine programmierbare Vorrichtung auf dem Kanister eines oder mehr von:
einer Radiofrequenzerkennungsvorrichtung (RFID);
einem Mikrochip;
einem Subscriber-Identity-Module-(SIM)-Chip;
einer Smartcard;
einem Barcode; und
einem Magnetstreifen
umfasst.

52. Ein Kanister gemäß irgendeinem der Ansprüche 48 bis 51, weiter umfassend Manipulationsvorbeugungsmittel, um Zugang zum Kanister zu verhindern, wenn die Befüllungs- oder Ausgaberegeln nicht erfüllt worden sind.

53. Ein Kanister gemäß irgendeinem der Ansprüche 48 bis 52, wobei der Kanister weiter Manipulationsentdeckungsmittel zum Nachweis von Manipulationsversuchen umfasst.

54. Ein Kanister gemäß Anspruch 53, wobei programmierbare Vorrichtung auf dem Kanister einen Manipulationslog unterhält, um eine Aufzeichnung über Manipulationsversuche zu führen.

55. Ein Kanister gemäß Anspruch 52, wobei das Manipulationsvorbeugungsmittel eine Vorrichtung umfasst, die nur durch Einsatz eines Signals vom Ausgabe-/Füllapparat betätigt werden kann.

56. Ein Kanister gemäß Anspruch 53 oder Anspruch 54, wobei der Kanister konfiguriert ist für die Mitteilung der Entdeckung eines Manipulationsversuchs an einen zentralen Administrator oder eine andere befugte Einheit.

57. Ein Kanister gemäß irgendeinem der Ansprüche 48 bis 56, wobei das Ausgaberad angepasst ist zum Empfangen von ein oder mehr austauschbaren Randabschnitten mit verschiedenen Dosierungskompartimentkonfigurationen.

58. Ein Verfahren zur Ausgabe von Medikation an einen Patienten unter Verwendung eines Medikationsausgabesystems gemäß irgendeinem der Ansprüche 1 bis 47.

## Revendications

1. Système de distribution de médicament comprenant :
un appareil de remplissage ;
un appareil de distribution ;
une cartouche rechargeable portable comportant une pluralité de compartiments de dose, la cartouche pouvant être montée de façon amovible sur l'appareil de remplissage et l'appareil de distribution et possédant un ou plusieurs dispositifs programmables configurés pour communiquer avec l'appareil de remplissage et de distribution ; et
des moyens de commande configurés pour commander le remplissage en médicament et la sortie de médicament d'une cartouche fixée à un appareil de remplissage/distribution selon les informations stockées par le ou les dispositifs programmables sur la cartouche ;
**caractérisé en ce que** l'appareil de remplissage est configuré pour remplir les compartiments de dose automatiquement avec le médicament selon une prescription du patient communiquée à l'appareil de remplissage.

2. Système de distribution de médicament selon la revendication 1, dans lequel la cartouche comprend un dispositif programmable pour chaque compartiment de dose.

3. Système de distribution de médicament selon la revendication 1 ou la revendication 2, dans lequel le ou les dispositifs programmables sont configurés pour stocker des informations sur la cartouche, y compris une ou plusieurs parmi :
une identité de cartouche ;
la configuration des compartiments de dose dans la cartouche ;
le nombre de compartiments de dose dans la cartouche ;
un médicament ou une pluralité de médicaments contenus dans un compartiment de dose ;
une heure à laquelle une dose a été distribuée ;
une heure à laquelle une dose doit être distribuée ; et
une identité d'un patient auquel la cartouche est destinée.

4. Système de distribution de médicament selon l'une quelconque des revendications précédentes, dans lequel des dispositifs programmables sur la cartouche comprennent un ou plusieurs parmi :
un dispositif d'identification par radiofréquence (RFID) ;
un microchip ;
une carte à puce ;
une puce de module d'identification de l'abonné (SIM) ;
un code à barres ; et
une bande magnétique.

5. Système de distribution de médicament selon l'une quelconque des revendications précédentes comprenant en outre des moyens d'inviolabilité sur la cartouche pour empêcher un accès non autorisé au médicament à l'intérieur de la cartouche.

6. Système de distribution de médicament selon la revendication 5, dans lequel la cartouche comprend en outre des moyens de détection de violation pour détecter des tentatives de violation.

7. Système de distribution de médicament selon la revendication 5 ou la revendication 6, dans lequel les moyens d'inviolabilité comprennent un dispositif utilisable uniquement par application d'un signal provenant de l'appareil de distribution/remplissage.

8. Système de distribution de médicament selon l'une quelconque des revendications 5 à 7, dans lequel un dispositif programmable sur la cartouche stocke les informations relatives à une tentative de violation.

9. Dispositif de distribution de médicament selon la revendication 8, dans lequel le dispositif programmable sur la cartouche comprend en outre un journal des violations destiné à enregistrer les changements de l'état de détection de violation.

10. Système de distribution de médicament selon l'une quelconque des revendications 5 à 9, dans lequel un dispositif programmable sur la cartouche est configuré pour communiquer la détection d'une tentative de violation à un appareil de distribution/remplissage ou à un service à distance.

11. Système de distribution de médicament selon l'une quelconque des revendications précédentes, dans lequel la cartouche comprend en outre un dispositif de suivi pour suivre l'emplacement de la cartouche.

12. Système de distribution de médicament selon l'une quelconque des revendications précédentes, dans lequel l'appareil de remplissage et l'appareil de distribution sont incorporés dans une seule unité.

13. Système de distribution de médicament selon l'une quelconque des revendications précédentes, dans lequel la cartouche comprend :
un corps de cartouche ; et
une roue de distribution présentant une pluralité de compartiments de dose, la roue de distribution pouvant être couplée de façon rotative au corps de cartouche ;
dans lequel la rotation de la roue de distribution par rapport au corps de cartouche est commandée par les moyens de commande pour faciliter le remplissage en médicament et la sortie de médicament des compartiments de dose à travers une ouverture dans le corps de cartouche.

14. Système de distribution de médicament selon l'une quelconque des revendications précédentes, dans lequel l'appareil de distribution comprend :
une ou plusieurs zones de distribution qui sont utilisables indépendamment sous le contrôle des moyens de commande pour faciliter le remplissage en médicament ou la sortie de médicament des compartiments de dose.

15. Système de distribution de médicament selon l'une quelconque des revendications précédentes, dans lequel un ou plusieurs parmi les appareils de remplissage et de distribution comprend(comprennent) en outre un lecteur de dispositif destiné à lire le ou les dispositifs programmables sur une ou plusieurs cartouches.

16. Système de distribution de médicament selon l'une quelconque des revendications précédentes, dans lequel un ou plusieurs parmi l'appareil de remplissage et l'appareil de distribution est(sont) en communication avec un service d'informations à distance.

17. Système de distribution de médicament selon la revendication 16, dans lequel l'appareil de distribution comprend en outre un composant de communication destiné à transférer les informations relatives au médicament distribué au service d'information à distance.

18. Système de distribution de médicament selon l'une quelconque des revendications 1 à 17, dans lequel la prescription du patient est communiquée à l'appareil de remplissage par :
une cartouche ;
une base de données centrale ;
un médecin à distance ;
un pharmacien ;
un hôpital ; ou
une autre entité autorisée à distribuer et/ou prescrire le médicament.

19. Système de distribution de médicament selon la revendication 18, dans lequel l'appareil de remplissage comprend en outre un dispositif de mesure de remplissage pour mesurer une quantité d'unités de médicament introduites dans un compartiment de dose d'une cartouche.

20. Système de distribution de médicament selon la revendication 19, dans lequel le dispositif de mesure de remplissage est en communication avec les moyens de commande pour le remplissage automatique en unités de médicament des compartiments de dose.

21. Système de distribution de médicament selon la revendication 19 ou la revendication 20, dans lequel le dispositif de mesure de remplissage comprend un ou plusieurs parmi:
un compteur ;
une graduation ;
oeil électronique ;
dispositif de reconnaissance optique ; et
une caméra ;
adapté pour quantifier les unités de médicament introduites dans le compartiment de dose.

22. Système de distribution de médicament selon l'une quelconque des revendications précédentes comprenant en outre une interface de facturation pour facturer automatiquement un patient, un employeur, un fournisseur de soins de santé, une compagnie d'assurance ou une autre entité facturable pour le médicament distribué par le système.

23. Système de distribution de médicament selon l'une quelconque des revendications précédentes, dans lequel l'appareil de remplissage comprend en outre une imprimante configurée pour imprimer une étiquette pouvant être apposée sur la cartouche, l'étiquette affichant des informations relatives à un régime posologique du patient.

24. Système de distribution de médicament selon la revendication 23, dans lequel l'imprimante est configurée pour imprimer automatiquement une nouvelle étiquette contenant de nouvelles informations lorsque le régime posologique du patient change.

25. Système de distribution de médicament comprenant :
un appareil de distribution ;
une cartouche portable rechargeable comportant une pluralité de compartiments de dose, la cartouche pouvant être montée de façon amovible sur l'appareil de distribution et possédant un ou plusieurs dispositifs programmables configurés pour communiquer avec l'appareil de distribution ; et
des moyens de commande configurés pour commander l'appareil de distribution pour faciliter la sortie de médicament d'un compartiment de dose selon les informations stockées par le ou les dispositifs programmables sur la cartouche ;
**caractérisé en ce que** les compartiments de dose sont remplis automatiquement avec le médicament selon une prescription du patient et le médicament est distribué à partir des compartiments de dose selon les informations stockées par le ou les dispositifs programmables sur la cartouche, lesdites informations se rapportant au régime posologique prédéterminé du patient.

26. Système de distribution de médicament selon la revendication 25, dans lequel la cartouche comprend un dispositif programmable pour chaque compartiment de dose.

27. Système de distribution de médicament selon la revendication 25 ou la revendication 26, dans lequel la cartouche comprend en outre des moyens d'inviolabilité pour empêcher un accès non autorisé au médicament à l'intérieur de la cartouche.

28. Système de distribution de médicament selon la revendication 27, dans lequel la cartouche comprend en outre des moyens de détection de violation destinés à détecter une tentative de violation.

29. Système de distribution de médicament selon la revendication 27 ou la revendication 28, dans lequel les moyens d'inviolabilité comprennent un dispositif utilisable uniquement par application d'un signal provenant de l'appareil de distribution/remplissage.

30. Dispositif de distribution de médicament selon la revendication 28, dans lequel dispositif programmable sur la cartouche comprend en outre un journal des violations destiné à enregistrer les changements de l'état de détection de violation.

31. Système de distribution de médicament selon l'une quelconque des revendications 28 à 30, dans lequel un dispositif programmable sur la cartouche est configuré pour communiquer la détection d'une tentative de violation à un appareil de distribution/remplissage et/ou à un service à distance.

32. Système de distribution de médicament selon l'une quelconque des revendications 25 à 31, dans lequel la cartouche comprend en outre un dispositif de suivi pour suivre l'emplacement de la cartouche.

33. Système de distribution de médicament selon l'une quelconque des revendications 25 à 32, dans lequel la cartouche comprend en outre ;
un corps de cartouche ; et
une roue de distribution présentant une pluralité de compartiments de dose, la roue de distribution pouvant être couplée de façon rotative au corps de cartouche ;
dans lequel la rotation de la roue de distribution par rapport au corps de cartouche est commandée par les moyens de commande pour faciliter la sortie de médicament d'un compartiment de dose à travers une ouverture dans le corps de cartouche.

34. Système de distribution de médicament selon l'une quelconque des revendications 25 à 33, dans lequel l'appareil de distribution comprend :
une ou plusieurs zones de distribution qui sont utilisables indépendamment sous le contrôle des moyens de commande pour faciliter la sortie de médicament des compartiments de dose.

35. Système de distribution de médicament selon l'une quelconque des revendications 25 à 34, dans lequel un dispositif d'identification programmable comprend un ou plusieurs parmi:
un dispositif d'identification par radiofréquence (RFID) ;
un microchip ;
une puce de module d'identification de l'abonné (SIM) ;
une carte à puce ;
un code à barres ; et
une bande magnétique.

36. Système de distribution de médicament selon l'une quelconque des revendications 25 à 35, dans lequel l'appareil de distribution comprend en outre un lecteur de dispositif destiné à lire le ou les dispositifs programmables sur la cartouche.

37. Système de distribution de médicament selon l'une quelconque des revendications 25 à 36, comprenant en outre une interface de facturation pour facturer automatiquement un patient, un employeur, un fournisseur de soins de santé, une compagnie d'assurance ou une autre entité facturable pour le médicament distribué à partir du système.

38. Système de distribution de médicament selon l'une quelconque des revendications 1 à 35, dans lequel l'appareil de distribution comprend en outre des moyens d'affichage pour indiquer visuellement les informations comprenant un ou plusieurs parmi l'heure actuelle, l'heure à laquelle la prochaine dose doit être distribuée, l'identité du patient, l'identité du médicament, les coordonnées de contact en cas d'urgence et les informations de dose.

39. Système de distribution de médicament selon l'une quelconque des revendications précédentes, configuré pour stocker électroniquement un enregistrement d'événements d'accès à la cartouche y compris, mais sans limitation, un identifiant d'une personne accédant à la cartouche, une heure à laquelle quelqu'un a accédé à la cartouche et l'endroit où l'accès s'est produit.

40. Système de distribution de médicament selon l'une quelconque des revendications précédentes, comprenant en outre un élément mobile pour séparer des unités de médicament distribuées de telle sorte qu'elles puissent être identifiées par un système de détection optique.

41. Système de distribution de médicament selon l'une quelconque des revendications précédentes, dans lequel l'appareil de distribution comprend en outre un système d'alarme pour alerter un patient qu'une dose de médicament doit être distribuée.

42. Système de distribution de médicament selon la revendication 41, dans lequel le système d'alarme émet une alerte lorsqu'un patient n'a pas distribué un médicament prescrit dans une tranche de temps prédéterminée.

43. Système de distribution de médicament selon la revendication 42, dans lequel il existe une pluralité de tranches de temps prédéterminées et de niveaux d'alerte correspondants, et dans lequel l'écoulement d'une tranche de temps plus longue fait monter le niveau d'alerte à un niveau d'alerte supérieur.

44. Système de distribution de médicament selon la revendication 41, dans lequel le système d'alarme exécute une procédure d'alerte selon un niveau d'alerte, la procédure d'alerte comprenant une ou plusieurs parmi :
émission d'une alarme sonore ;
transmission d'un message à un dispositif de communication du patient ;
transmission d'un message à un aidant du patient ;
transmission d'un message à l'établissement de santé du patient ;
transmission d'un message au médecin du patient ; et
transmission d'une alerte à une organisation d'urgence.

45. Système de distribution de médicament selon l'une quelconque des revendications 41 à 44, dans lequel le système d'alarme peut être utilisé par un patient pour envoyer une alerte de détresse à un ou plusieurs parmi :
les moyens de contrôle ;
un aidant du patient ;
un établissement de santé ; et
un médecin.

46. Système de distribution de médicament selon l'une quelconque des revendications précédentes, dans lequel les moyens de commande commandent la sortie du médicament vers un patient selon une ou plusieurs règles de distribution prédéterminées stockées par un dispositif programmable sur la cartouche, les règles comprenant une ou plusieurs parmi :
le patient a-t-il été correctement identifié ?
la cartouche a-t-elle été correctement identifiée ?
le médicament devant être distribué est-il correct ?
l'heure de distribution du médicament est-elle correcte ? la cartouche est-elle exempte de tentatives de violation ? et
le médicament n'est-il pas contre-indiqué avec d'autres médicaments prescrits ?

47. Système de distribution de médicament selon l'une quelconque des revendications précédentes, dans lequel les moyens de commande actualisent automatiquement les informations de dose stockées par le dispositif programmable sur la cartouche.

48. Cartouche destinée à être utilisée avec un système de distribution de médicament, la cartouche comprenant :
un corps de cartouche présentant une ouverture de distribution ;
une roue de distribution présentant une pluralité de compartiments de dose et pouvant être couplée de façon rotative au corps de cartouche ; et
un ou plusieurs dispositifs programmables configurés pour communiquer avec appareil pour le remplissage en médicament ou la distribution du médicament ;
dans lequel le médicament est introduit dans ou distribué à partir de la cartouche en la fixant à un appareil de remplissage et/ou de distribution possédant des moyens de commande, et dans lequel les moyens de commande commandent la rotation de la roue de distribution par rapport au corps de cartouche pour faciliter le remplissage en médicament et la sortie de médicament des compartiments de dose selon les informations stockées sur le ou les dispositifs programmables sur la cartouche ;
**caractérisé en ce que** les compartiments de dose sont remplis automatiquement en médicament selon la prescription du patient et les informations stockées sur le ou les dispositifs programmables sur la cartouche se rapportent à un régime posologique prédéterminé du patient.

49. Cartouche selon la revendication 48, comprenant en outre un dispositif programmable pour chaque compartiment de dose dans la roue de distribution.

50. Cartouche selon la revendication 48 ou la revendication 49, dans laquelle le ou les dispositifs programmables stockent des informations comprenant un ou plusieurs parmi l'identité de la cartouche, la configuration des compartiments de dose dans la cartouche, le nombre de sections de bord externes dans la roue de distribution, le nombre de compartiments de dose dans la cartouche, un médicament ou une pluralité de médicaments contenus dans un compartiment de dose, une heure à laquelle une dose a été distribuée ou doit être distribuée, et une identité d'un patient auquel la cartouche est destinée.

51. Cartouche selon la revendication 49, dans laquelle un dispositif programmable sur la cartouche comprend un ou plusieurs parmi les éléments suivants :
un dispositif d'identification par radiofréquence (RFID) ;
un microchip ;
une puce de module d'identification de l'abonné (SIM) ;
une carte à puce ;
un code à barres ; et
une bande magnétique.

52. Cartouche selon l'une quelconque des revendications 48 à 51, comprenant en outre des moyens d'inviolabilité pour empêcher l'accès à la cartouche lorsque les règles de remplissage ou de distribution n'ont pas été satisfaites.

53. Cartouche selon l'une quelconque des revendications 48 à 52, dans laquelle la cartouche comprend en outre des moyens de détection de violation pour détecter des tentatives de violation.

54. Cartouche selon la revendication 53, dans laquelle le dispositif programmable sur la cartouche présente un journal des violations pour conserver un enregistrement des tentatives de violation.

55. Cartouche selon la revendication 52, dans laquelle les moyens d'inviolabilité comprennent un dispositif utilisable uniquement par application d'un signal provenant de l'appareil de distribution/remplissage.

56. Cartouche selon la revendication 53 ou la revendication 54, dans laquelle la cartouche est configurée pour communiquer la détection d'une tentative de violation à un administrateur central ou une autre entité autorisée.

57. Cartouche selon l'une quelconque des revendications 48 à 56, dans laquelle la roue de distribution est adaptée pour recevoir une ou plusieurs parties de bord interchangeables présentant diverses configurations de compartiment de dose.

58. Procédé de distribution de médicament à un patient utilisant un système de distribution de médicament selon l'une quelconque des revendications 1 à 47.
